# EUROPEAN PATENT SPECIFICATION

(11) **EP 3 773 656 B1**
(45) Date of publication and mention of the grant of the patent: **28.10.2026**
(21) Application number: 19719403.8
(22) Date of filing: 08.04.2019
(51) Int. Cl.: C12N 15/00, C07K 14/495, A61K 38/00, A61K 38/18, C07K 14/475

(54) **GROWTH DIFFERENTIATION FACTOR 15 FUSION PROTEINS**
WACHSTUMSDIFFERENZIERUNGSFAKTOR-15-FUSIONSPROTEINE
PROTÉINES DE FUSION DU FACTEUR DE DIFFÉRENCIATION DE CROISSANCE 15

(30) Priority: 09.04.2018 US 201862655108 P
(43) Date of publication of application: 17.02.2021
(73) Proprietor: Amgen Inc., Thousand Oaks, CA 91320-1799 (US)
(72) Inventor: XIONG, YuMei, Thousand Oaks, California 91320-1799 (US); WALKER, Kenneth William, Thousand Oaks, California 91320-1799 (US); VENIANT ELLISON, Murielle Marie, Thousand Oaks, California 91320-1799 (US)
(74) Representative: Grünecker Patent- und Rechtsanwälte PartG mbB
(86) International application number: PCT/US2019/026369
(87) International publication number: WO 2019/199685

(56) References cited:
- WO-A1-2016/069921
- WO-A2-2014/120619
- US-A1- 2014 213 511
- US-B2- 9 714 276
- DATABASE USPTO Proteins [online] 15 August 2016 (2016-08-15), "Sequence 30 from patent US 9161966.", retrieved from EBI accession no. USPOP:AOC38718 Database accession no. AOC38718
- DATABASE Geneseq [online] 31 May 2018 (2018-05-31), "Human mature mutant GDF15 protein w5, SEQ ID 68.", retrieved from EBI accession no. GSP:BFE54584 Database accession no. BFE54584

## Description

### SEQUENCE LISTING

The present application is being filed along with a Sequence Listing in electronic format. The Sequence Listing is provided as a file entitled A-2239-WO-PCT_SeqList_ST25.txt, created March 15, 2019, which is 109 kb in size.

### FIELD OF THE INVENTION

The instant disclosure relates to GDF15 molecules, such as GDF15 fusion proteins, compositions thereof, and methods for making and using such proteins.

### BACKGROUND

Growth differentiation factor 15 (GDF15), also referred to as macrophage inhibitory cytokine 1 (MIC1) (Bootcov MR, 1997, Proc Natl Acad Sci 94:11514-9), placental bone morphogenetic factor (PLAB) (Hromas R 1997, Biochim Biophys Acta. 1354:40-4), placental transforming growth factor beta (PTGFB) (Lawton LN 1997, Gene. 203:17-26), prostate derived factor (PDF) (Paralkar VM 1998, J Biol Chem. 273:13760-7), and nonsteroidal antiinflammatory drug-activated gene (NAG-1) (Baek SJ 2001, J Biol Chem. 276: 33384-92), is a secreted protein that circulates in plasma as an ~25 kDa homodimer. GDF15 binds to GDNF family receptor α-like (GFRAL) with high affinity. GDF15-induced cell signaling is believed to require the interaction of GFRAL with the coreceptor RET. WO2016/069921 provides complexes, in which a GDF15 polypeptide is comprised.

GDF15 has been linked to multiple biological activities. Elevated GDF15 has been shown to be correlated with weight loss and administration of GDF15 has been shown to reduce food intake and body weight. Accordingly, there is a need for efficacious GDF15 molecules that can be administered as a therapeutic. The present disclosure provides GDF15 molecules that meets this need and provide related advantages.

### SUMMARY

Provided herein is a GDF15-Fc fusion protein as defined in claim 1 comprising a GDF15 region joined to an Fc region via a linker, wherein the GDF15 region comprises the amino acid sequence of SEQ ID NO: 16 or SEQ ID NO: 18, wherein the linker is a (G₄S)ₙ or (G₄Q)ₙ linker, wherein n is greater than 0..

Further embodiments are defined in the claims.

Also provided herein are dimers and tetramers comprising the fusion proteins - see claim 8.

Further provided are medical uses and pharmaceutical compositions as defined in the claims.

### BRIEF DESCRIPTION OF THE DRAWINGS

**Figure 1** is a graph showing the effect on the body weight of cynomologus monkeys dosed with vehicle, 3 mg/kg of the positive control FGF21-Fc, 1.5 mg/kg of scFc-GDF15, or 1.5 mg/kg of the dimer FcΔ10(-)-(G4S)4-GDF15:FcΔ10(+,K) weekly for six weeks, followed by a five-week washout.
**Figure 2** is a plot showing the effect on the triglyceride levels of cynomologus monkeys dosed with vehicle, 3 mg/kg of the positive control FGF21-Fc, 1.5 mg/kg of scFc-GDF15, or 1.5 mg/kg of the dimer FcΔ10(-)-(G4S)4-GDF15:FcΔ10(+,K) weekly for six weeks, followed by a five-week washout.
**Figure 3** shows the profile of FcΔ10(-)-(G4S)4-GDF15 after cation exchange.
**Figure 4** is a peptide map of FcΔ10(+)-(G4)-GDF15.
**Figure 5** is a graph showing the effect on food intake in mice as a function of dose of the dimers FcΔ10(-)-GDF15(Δ3):FcΔ10(+,K) (SEQ ID NOs: 41 and 32); FcΔ10(-)-GDF15(N3D):FcΔ10(+,K) (SEQ ID NOs: 42 and 32); FcΔ10(-,CC)-GDF15(Δ3):FcΔ10(+,K,CC) (SEQ ID NOs: 43 and 34); FcΔ10(-,CC)-GDF15(N3D):FcΔ10(+,K,CC) (SEQ ID NOs: 44 and 34)) and FcΔ10(-)-(G4S)4-GDF15:FcΔ10(+,K) (SEQ ID NOs:39 and 32).
**Figure 6** is a graph of the serum concentration of FcΔ10(-)-GDF15(Δ3) (SEQ ID NO: 41); FcΔ10(-)-GDF15(N3D) (SEQ ID NO: 42); FcΔ10(-,CC)-GDF15(Δ3) (SEQ ID NO: 43); and FcΔ10(-,CC)-GDF15(N3D) (SEQ ID NO: 44)) as a function of time in mice.
**Figure 7** is a graph showing the effect on the body weight of cynomologus monkeys dosed with vehicle, 3 mg/kg of the positive control FGF21-Fc, 0.5 mg/kg or 3.0 mg/kg of FcΔ16(-,CC)-GDF15(Δ3/D5E):FcΔ16(+,K,CC) (SEQ ID NOs: 45 and 35), 0.5 mg/kg or 3.0 mg/kg of FcΔ16(-,CC)-GDF15(N3Q/D5E):FcΔ16(+,K,CC)) (SEQ ID NOs: 46 and 35) or 0.5 mg/kg or 3.0 mg/kg of FcΔ16(-)-GDF15(N3Q/D5E):FcΔ16(+,K) (SEQ ID NOs: 47 and 36) weekly for four weeks, followed by a four-week washout.
**Figure 8** is a graph showing the effect on the body weight of cynomologus monkeys dosed with vehicle, 1.5 mg/kg of FcΔ10(-)-(G4S)4-GDF15:FcΔ10(+,K) (SEQ ID NOs: 39 and 32), 1.5 mg/kg of FcΔ16(-)-(G4Q)4-GDF15(N3Q):FcΔ16(+,K) (SEQ ID NOs: 49 and 36); 1.5 mg/kg of FcΔ16(-)-(G4Q)4-GDF15(N3Q/D5E):FcΔ16(+,K) (SEQ ID NOs: 50 and 36), 1.5 mg/kg of FcΔ16(-)-G4S-GDF15(N3Q/D5E):FcΔ16(+,K) (SEQ ID NOs: 54 and 36), or 1.5 mg/kg of FcΔ10(-,L234A/L235A)-(G4Q)4-GDF15(N3Q/D5E):FcΔ10(+,K,L234A/L235A) (SEQ ID NOs: 57 and 37) weekly for two weeks.
**Figure 9** is a graph of food intake as a function of dose in ob/ob mice administered FcΔ16(-)-(G4Q)4-GDF15(N3Q/D5E):FcΔ16(+,K) (SEQ ID NOs: 50 and 36).
**Figure 10** is a graph of food intake as a function of dose in ob/ob mice administered FcΔ10(-,L234A/L235A)-(G4Q)4-GDF15(N3Q/D5E):FcΔ10(+,K,L234A/L235A) (SEQ ID NOs: 57 and 37).

### DETAILED DESCRIPTION

Provided herein are fusion proteins comprising a GDF15 region, wherein the GDF15 region comprises the amino acid sequence of SEQ ID NO: 16 or SEQ ID NO: 18: dimers or tetramers comprising such fusion proteins and corresponding medical uses and pharmaceutical compositions.

Illustrated herein is wild type GDF15. Both the human and murine GDF15 have a signal peptide and prodomain. The nucleotide sequence for full-length human GDF15 is:

atgcccgggc aagaactcag gacggtgaat ggctctcaga tgctcctggt gttgctggtg ctctcgtggc tgccgcatgg
gggcgccctg tctctggccg aggcgagccg cgcaagtttc ccgggaccct cagagttgca ctccgaagac tccagattcc
gagagttgcg gaaacgctac gaggacctgc taaccaggct gcgggccaac cagagctggg aagattcgaa caccgacctc
gtcccggccc ctgcagtccg gatactcacg ccagaagtgc ggctgggatc cggcggccac ctgcacctgc gtatctctcg
ggccgccctt cccgaggggc tccccgaggc ctcccgcctt caccgggctc tgttccggct gtccccgacg gcgtcaaggt
cgtgggacgt gacacgaccg ctgcggcgtc agctcagcct tgcaagaccc caggcgcccg cgctgcacct gcgactgtcg
ccgccgccgt cgcagtcgga ccaactgctg gcagaatctt cgtccgcacg gccccagctg gagttgcact tgcggccgca
agccgccagg gggcgccgca gagcgcgtgc gcgcaacggg gaccactgtc cgctcgggcc cgggcgttgc tgccgtctgc
acacggtccg cgcgtcgctg gaagacctgg gctgggccga ttgggtgctg tcgccacggg aggtgcaagt gaccatgtgc
atcggcgcgt gcccgagcca gttccgggcg gcaaacatgc acgcgcagat caagacgagc ctgcaccgcc tgaagcccga
cacggtgcca gcgccctgct gcgtgcccgc cagctacaat cccatggtgc tcattcaaaa gaccgacacc ggggtgtcgc
tccagaccta tgatgacttg ttagccaaag actgccactg catatga (SEQ ID NO: 1)

The amino acid sequence for full-length human GDF15 (308 amino acids) is:

MPGQELRTVNGSQMLLVLLVLSWLPHGGALSLAEASRASFPGPSELHSEDSRFRELR
KRYEDLLTRLRANQSWEDSNTDLVPAPAVRILTPEVRLGSGGHLHLRISRAALPEGLP
EASRLHRALFRLSPTASRSWDVTRPLRRQLSLARPQAPALHLRLSPPPSQSDQLLAESS
SARPQLELHLRPQAARGRRRARARNGDHCPLGPGRCCRLHTVRASLEDLGWADWV
LSPREVQVTMCIGACPSQFRAANMHAQIKTSLHRLKPDTVPAPCCVPASYNPMVLIQ
KTDTGVSLQTYDDLLAKDCHCI (SEQ ID NO: 2)

The nucleotide sequence for human GDF15 without its signal sequence is:

ctgtctctgg ccgaggcgag ccgcgcaagt ttcccgggac cctcagagtt gcactccgaa gactccagat tccgagagtt
gcggaaacgc tacgaggacc tgctaaccag gctgcgggcc aaccagagct gggaagattc gaacaccgac ctcgtcccgg
cccctgcagt ccggatactc acgccagaag tgcggctggg atccggcggc cacctgcacc tgcgtatctc tcgggccgcc
cttcccgagg ggctccccga ggcctcccgc cttcaccggg ctctgttccg gctgtccccg acggcgtcaa ggtcgtggga
cgtgacacga ccgctgcggc gtcagctcag ccttgcaaga ccccaggcgc ccgcgctgca cctgcgactg

tcgccgccgc cgtcgcagtc ggaccaactg ctggcagaat cttcgtccgc acggccccag ctggagttgc acttgcggcc
gcaagccgcc agggggcgcc gcagagcgcg tgcgcgcaac ggggaccact gtccgctcgg gcccgggcgt
tgctgccgtc tgcacacggt ccgcgcgtcg ctggaagacc tgggctgggc cgattgggtg ctgtcgccac gggaggtgca
agtgaccatg tgcatcggcg cgtgcccgag ccagttccgg gcggcaaaca tgcacgcgca gatcaagacg agcctgcacc
gcctgaagcc cgacacggtg ccagcgccct gctgcgtgcc cgccagctac aatcccatgg tgctcattca aaagaccgac
accggggtgt cgctccagac ctatgatgac ttgttagcca aagactgcca ctgcatatga (SEQ ID NO: 3)

The amino acid sequence for human GDF15 without its 29 amino acid signal sequence (279 amino acids) is:

LSLAEASRASFPGPSELHSEDSRFRELRKRYEDLLTRLRANQSWEDSNTDLVPAPAVR
ILTPEVRLGSGGHLHLRISRAALPEGLPEASRLHRALFRLSPTASRSWDVTRPLRRQLS
LARPQAPALHLRLSPPPSQSDQLLAESSSARPQLELHLRPQAARGRRRARARNGDHCP
LGPGRCCRLHTVRASLEDLGWADWVLSPREVQVTMCIGACPSQFRAANMHAQIKTS
LHRLKPDTVPAPCCVPASYNPMVLIQKTDTGVSLQTYDDLLAKDCHCI (SEQ ID NO:
4)

The nucleotide sequence for human GDF15 without its signal peptide or prodomain is:

gcgcgcaacggggaccactgtccgctcgggcccgggcgttgctgccgtctgcacacggtccgcgcgtcgctggaagacctgggct
gggccgattgggtgctgtcgccacgggaggtgcaagtgaccatgtgcatcggcgcgtgcccgagccagttccgggcggcaaacatg
cacgcgcagatcaagacgagcctgcaccgcctgaagcccgacacggtgccagcgccctgctgcgtgcccgccagctacaatcccat
ggtgctcattcaaaagaccgacaccggggtgtcgctccagacctatgatgacttgttagccaaagactgccactgcatatga (SEQ
ID NO: 5)

The amino acid sequence for human GDF15 without its signal peptide or pro-domain the active domain of GDF15 of 112 amino acids) is:

ARNGDHCPLGPGRCCRLHTVRASLEDLGWADWVLSPREVQVTMCIGACPSQFRAAN
MHAQIKTSLHRLKPDTVPAPCCVPASYNPMVLIQKTDTGVSLQTYDDLLAKDCHCI
(SEQ ID NO: 6)

The nucleotide sequence for full-length murine GDF15 is:

atggccccgc ccgcgctcca ggcccagcct ccaggcggct ctcaactgag gttcctgctg ttcctgctgc tgttgctgct
gctgctgtca tggccatcgc agggggacgc cctggcaatg cctgaacagc gaccctccgg ccctgagtcc caactcaacg
ccgacgagct acggggtcgc ttccaggacc tgctgagccg gctgcatgcc aaccagagcc gagaggactc
gaactcagaa ccaagtcctg acccagctgt ccggatactc agtccagagg tgagattggg gtcccacggc cagctgctac
tccgcgtcaa ccgggcgtcg ctgagtcagg gtctccccga agcctaccgc gtgcaccgag cgctgctcct gctgacgccg
acggcccgcc cctgggacat cactaggccc ctgaagcgtg cgctcagcct ccggggaccc cgtgctcccg cattacgcct
gcgcctgacg ccgcctccgg acctggctat gctgccctct ggcggcacgc agctggaact gcgcttacgg
gtagccgccg gcagggggcg ccgaagcgcg catgcgcacc caagagactc gtgcccactg ggtccggggc gctgctgtca
cttggagact gtgcaggcaa ctcttgaaga cttgggctgg agcgactggg tgctgtcccc gcgccagctg cagctgagca
tgtgcgtggg cgagtgtccc cacctgtatc gctccgcgaa cacgcatgcg cagatcaaag cacgcctgca tggcctgcag
cctgacaagg tgcctgcccc gtgctgtgtc ccctccagct acaccccggt ggttcttatg cacaggacag acagtggtgt
gtcactgcag acttatgatg acctggtggc ccggggctgc cactgcgctt ga (SEQ ID NO: 7)

The amino acid sequence for full-length murine GDF15 (303 amino acids) is:

MAPPALQAQPPGGSQLRFLLFLLLLLLLLSWPSQGDALAMPEQRPSGPESQLNADEL
RGRFQDLLSRLHANQSREDSNSEPSPDPAVRILSPEVRLGSHGQLLLRVNRASLSQGL
PEAYRVHRALLLLTPTARPWDITRPLKRALSLRGPRAPALRLRLTPPPDLAMLPSGGT
QLELRLRVAAGRGRRSAHAHPRDSCPLGPGRCCHLETVQATLEDLGWSDWVLSPRQ
LQLSMCVGECPHLYRSANTHAQIKARLHGLQPDKVPAPCCVPSSYTPVVLMHRTDS
GVSLQTYDDLVARGCHCA (SEQ ID NO: 8)

The nucleotide sequence for murine GDF15 without its signal sequence is:

tcgcagggggacgccctggcaatgcctgaacagcgaccctccggccctgagtcccaactcaacgccgacgagctacggggtcgctt
ccaggacctgctgagccggctgcatgccaaccagagccgagaggactcgaactcagaaccaagtcctgacccagctgtccggatac
tcagtccagaggtgagattggggtcccacggccagctgctactccgcgtcaaccgggcgtcgctgagtcagggtctccccgaagcct
accgcgtgcaccgagcgctgctcctgctgacgccgacggcccgcccctgggacatcactaggcccctgaagcgtgcgctcagcctc
cggggaccccgtgctcccgcattacgcctgcgcctgacgccgcctccggacctggctatgctgccctctggcggcacgcagctgga
actgcgcttacgggtagccgccggcagggggcgccgaagcgcgcatgcgcacccaagagactcgtgcccactgggtccggggcg
ctgctgtcacttggagactgtgcaggcaactcttgaagacttgggctggagcgactgggtgctgtccccgcgccagctgcagctgagc
atgtgcgtgggcgagtgtccccacctgtatcgctccgcgaacacgcatgcgcagatcaaagcacgcctgcatggcctgcagcctgac
aaggtgcctgccccgtgctgtgtcccctccagctacaccccggtggttcttatgcacaggacagacagtggtgtgtcactgcagacttat
gatgacctggtggcccggggctgccactgcgcttga (SEQ ID NO: 9)

The amino acid sequence for murine GDF15 without its 32 amino acid signal sequence (271 amino acids) is:

SQGDALAMPEQRPSGPESQLNADELRGRFQDLLSRLHANQSREDSNSEPSPDPAVRIL
SPEVRLGSHGQLLLRVNRASLSQGLPEAYRVHRALLLLTPTARPWDITRPLKRALSLR
GPRAPALRLRLTPPPDLAMLPSGGTQLELRLRVAAGRGRRSAHAHPRDSCPLGPGRC
CHLETVQATLEDLGWSDWVLSPRQLQLSMCVGECPHLYRSANTHAQIKARLHGLQP
DKVPAPCCVPSSYTPVVLMHRTDSGVSLQTYDDLVARGCHCA (SEQ ID NO: 10)

The nucleotide sequence for murine GDF15 without its signal sequence or pro-domain is:

agcgcgcatgcgcacccaagagactcgtgcccactgggtccggggcgctgctgtcacttggagactgtgcaggcaactcttgaagac
ttgggctggagcgactgggtgctgtccccgcgccagctgcagctgagcatgtgcgtgggcgagtgtccccacctgtatcgctccgcg
aacacgcatgcgcagatcaaagcacgcctgcatggcctgcagcctgacaaggtgcctgccccgtgctgtgtcccctccagctacacc
ccggtggttcttatgcacaggacagacagtggtgtgtcactgcagacttatgatgacctggtggcccggggctgccactgcgcttga
(SEQ ID NO: 11)

The amino acid sequence for murine GDF15 without its signal peptide or prodomain (active domain of 115 amino acids) is:

SAHAHPRDSCPLGPGRCCHLETVQATLEDLGWSDWVLSPRQLQLSMCVGECPHLYR
SANTHAQIKARLHGLQPDKVPAPCCVPSSYTPVVLMHRTDSGVSLQTYDDLVARGC
HCA (SEQ ID NO: 12)

The GDF15 molecule may comprise a GDF15 region comprising an active domain of GDF15, *e.g.,* GDF15 without its signal peptide or pro-domain. The GDF15 region may comprise the amino acid sequence of SEQ ID NO: 6 or 12. The GDF15 region may comprise a GDF15 sequence with one or more mutations, such as at least one mutation in the active domain of GDF15. The mutation or mutations may not reduce or eliminate the activity of GDF15. The GDF15 region may comprise a mutation in the active domain of human GDF15. The mutation may be a deletion of the first three amino acids of the active domain, such as "GDF15(Δ3)" which is an active domain of human GDF15 in which the first three amino acids removed *(i.e.,* SEQ ID NO: 13).

The GDF15 region may comprise a mutation of the asparagine at position 3 (N3) of the active domain of human GDF15 (SEQ ID NO: 6). An N3 mutation can refer to the mutation of the asparagine residue at position 3 of SEQ ID NO: 6 or the mutation of an asparagine residue corresponding to the asparagine at position 3 of SEQ ID NO: 6 in a GDF15 amino acid sequence. The asparagine at position 3 may mutated to glutamine (N3Q) or aspartate (N3D). In some embodiments, the GDF15 molecule comprises a GDF15 region of GDF15(D5E), which has the amino acid sequence of SEQ ID NO: 16.

In yet other embodiments, the GDF15 region comprises a combination of N3 and D5 mutations, e.g., GDF15(N3Q/D5E). In one claimed alternative, the GDF15 region comprises the amino acid sequence of SEQ ID NO: 18.

Table 1 provides examples of GDF15 regions that can be used in the GDF15 molecules. SEQ ID NO: 16 or 18 is comprised in the fusion proteins of the present invention.

**Table 1 - GDF15 Regions**

| **SEQ ID NO:** | **Designation** | **Sequence** |
|---|---|---|
| 6 | GDF15 | ARNGDHCPLGPGRCCRLHTVRASLEDLGWADWVLS PREVQVTMCIGACPSQFRAANMHAQIKTSLHRLKPD TVPAPCCVPASYNPMVLIQKTDTGVSLQTYDDLLAK DCHCI |
| 13 | GDF15(Δ3) | GDHCPLGPGRCCRLHTVRASLEDLGWADWVLSPRE VQVTMCIGACPSQFRAANMHAQIKTSLHRLKPDTVP APCCVPASYNPMVLIQKTDTGVSLQTYDDLLAKDC HCI *First third amino acids at N-terminus of GDF15 sequence (SEQ ID NO: 6) is deleted in this GDF15 region.* |
| 14 | GDF15(N3Q) | ARQGDHCPLGPGRCCRLHTVRASLEDLGWADWVLS PREVQVTMCIGACPSQFRAANMHAQIKTSLHRLKPD TVPAPCCVPASYNPMVLIQKTDTGVSLQTYDDLLAK DCHCI *Underlined and bolded residue is N3Q mutation.* |
| 15 | GDF15(N3D) | ARDGDHCPLGPGRCCRLHTVRASLEDLGWADWVLS PREVQVTMCIGACPSQFRAANMHAQIKTSLHRLKPD |
| | | TVPAPCCVPASYNPMVLIQKTDTGVSLQTYDDLLAK DCHCI *Underlined and bolded residue is N3D mutation.* |
| 16 | GDF15(D5E) | ARNGEHCPLGPGRCCRLHTVRASLEDLGWADWVLS PREVQVTMCIGACPSQFRAANMHAQIKTSLHRLKPD TVPAPCCVPASYNPMVLIQKTDTGVSLQTYDDLLAK DCHCI *Underlined and bolded residue is D5E mutation.* |
| 17 | GDF15(Δ3/D5E) | GEHCPLGPGRCCRLHTVRASLEDLGWADWVLSPRE VQVTMCIGACPSQFRAANMHAQIKTSLHRLKPDTVP APCCVPASYNPMVLIQKTDTGVSLQTYDDLLAKDC HCI *First third amino acids at N-terminus of GDF15 sequence (SEQ ID NO: 6) is deleted in this GDF15 region; underlined and bolded residue is D5E mutation (position in reference to wild-type GDF15 sequence of SEQ ID NO: 6).* |
| 18 | GDF15(N3Q/D5E) | ARQGEHCPLGPGRCCRLHTVRASLEDLGWADWVLS PREVQVTMCIGACPSQFRAANMHAQIKTSLHRLKPD TVPAPCCVPASYNPMVLIQKTDTGVSLQTYDDLLAK DCHCI *Underlined and bolded residues are N3Q and D5E mutations.* |

According to the claims, the Fc is fused to the GDF15 molecule via a linker The linker is a (G4S)n or (G4Q)n linker, wherein n is greater than 0. In some embodiments, n is greater than or equal to 2, such as 3, 4, 5, 6, 7, or 8. In some embodiments, the linker comprises the amino acid sequence of SEQ ID NO: 19, 20, 21, 22, 24, or 25 as shown in Table 2.

**Table 2 - Linkers**

| **SEQ ID NO:** | **Designation** | **Sequence** |
|---|---|---|
| 19 | G4S | GGGGS |
| 20 | (G4S)2 | GGGGSGGGGS |
| 21 | (G4S)4 | GGGGSGGGGSGGGGSGGGGS |
| 22 | (G4S)8 | GGGGSGGGGSGGGGSGGGGSGGGGSGGGGSGGG GSGGGGS |
| 23 | G4 | GGGG |
| 24 | G4Q | GGGGQ |
| 25 | (G4Q)4 | GGGGQGGGGQGGGGQGGGGQ |
| 58 | G4A | GGGGA |

The GDF15 molecule comprises an Fc region. The Fc region can comprise or be derived from the Fc domain of a heavy chain of an antibody. In some embodiments, the Fc region may comprise an Fc domain with a mutation, such as a charged pair mutation, a mutation in a glycosylation site or the inclusion of an unnatural amino acid. The Fc region can be derived from a human IgG constant domain of IgG1, IgG2, IgG3 or IgG4. In some embodiments, the Fc region comprises the constant domain of an IgA, IgD, IgE, and IgM heavy chain.

In some embodiments, the Fc region comprises an Fc domain with a charged pair mutation. By introducing a mutation resulting in a charged Fc region, the GDF15 molecule can dimerize with a corresponding Fc molecule having the opposite charge. For example, an aspartate-to-lysine mutation (E356K, wherein 356 is the position using EU numbering, and corresponds to the positions as noted in Tables 3-5) and a glutamate-to-lysine mutation (D399K wherein 399 is the position using EU numbering, and corresponds to positions as noted in Tables 3-5) can be introduced into the Fc region that is joined to a GDF15 region, optionally via a linker, resulting in a positively charged Fc region for the GDF15 molecule. Lysine-to-aspartate mutations (K392D, K409D; wherein 392 and 409 are the positions using EU numbering and corresponds to the positions as noted in Tables 3-5) can be introduced into an Fc domain of a separate molecule, resulting in a negatively charged Fc molecule. The aspartate residues in the negatively charged Fc molecule can associate with the lysine residues of the positively charged Fc region of the GDF15 molecule through electrostatic force, facilitating formation of Fc heterodimers between the Fc region of the GDF15 molecule and the Fc molecule, while reducing or preventing formation of Fc homodimers between the Fc regions of the GDF15 molecules or between Fc molecules.

In some embodiments, one or more lysine-to-aspartate mutations (K392D, K409D) are introduced into the Fc region that is joined to a GDF15 region, optionally via a linker and an aspartate-to-lysine mutation (E356K) and a glutamate-to-lysine mutation (D399K) is introduced into the Fc domain of another molecule. The aspartate residues in the Fc region of the GDF15 molecule can associate with the lysine residues of the Fc molecule through electrostatic force, facilitating formation of Fc heterodimers between the Fc region of the GDF15 molecule and the Fc molecule, and reducing or preventing formation of Fc homodimers between the Fc regions of the GDF15 molecules or between Fc molecules.

In some embodiments, the GDF15 molecule comprises an Fc region comprising an Fc domain with a mutated hinge region. In some embodiments, the Fc domain comprises a deletion in the hinge. In some embodiments, ten amino acids from the hinge are deleted, e.g., FcΔ10. In other embodiments, sixteen amino acids from the hinge are deleted, e.g., FcΔ16. In some embodiments, the Fc domain comprises a hinge deletion (e.g., FcΔ10 or FcΔ16) and a charged pair mutation, such that the Fc domain is positively or negatively charged. For example, the Fc domain can comprise a ten-amino acid deletion in the hinge and lysine-to-aspartate mutations (K392D, K409D), such as FcΔ10(-). In another embodiment, the Fc domain can comprise a ten-amino acid deletion in the hinge and an aspartate-to-lysine mutation (E356K) and a glutamate-to-lysine mutation (D399K), such as an FcΔ10(+). In another embodiment, the Fc domain can comprise a sixteen-amino acid deletion in the hinge and lysine-to-aspartate mutations (K392D, K409D), such as FcΔ16(-). In another embodiment, the Fc domain can comprise a sixteen- amino acid deletion in the hinge and an aspartate-to-lysine mutation (E356K) and a glutamate-to-lysine mutation (D399K), such as an FcΔ16(+).

In some embodiments, an Fc molecule comprising a hinge deletion and a charged pair mutation heterodimerizes with such a GDF15 molecule. For example, the Fc molecule can have a hinge deletion and charged pair mutation that complements the hinge deletion and charged pair mutation of the Fc region of a GDF15 molecule. For example, an Fc molecule can comprise an Fc domain with a ten-amino acid deletion in the hinge and lysine-to-aspartate mutations (K392D, K409D), such as FcΔ10(-), which can optionally comprise a C-terminal lysine (e.g., FcΔ10(-, K)). The Fc molecule can heterodimerize with a GDF15 molecule that comprises an FcΔ10(+). In another embodiment, the Fc molecule can comprise a ten-amino acid deletion in the hinge and an aspartate-to-lysine mutation (E356K) and a glutamate-to-lysine mutation (D399K), such as an FcΔ10(+), which can optionally comprise a C-terminal lysine (e.g., FcΔ10(+, K)). The Fc molecule can heterodimerize with a GDF15 molecule that comprises an FcΔ10(-). In another embodiment, the Fc molecule can comprise a sixteen-amino acid deletion in the hinge and lysine-to-aspartate mutations (K392D, K409D), such as FcΔ16(-), which can optionally comprise a C-terminal lysine (e.g., FcΔ16(-, K)). The Fc molecule which can heterodimerize with a GDF15 molecule that comprises an FcΔ16(+). In another embodiment, the Fc molecule can comprise a sixteen-amino acid deletion in the hinge and an aspartate-to-lysine mutation (E356K) and a glutamate-to-lysine mutation (D399K), such as an FcΔ16(+), which can optionally comprise a C-terminal lysine (e.g., FcΔ16(-, K)). The Fc molecule can heterodimerize with a GDF15 molecule that comprises an FcΔ16(-).

In some embodiments, the Fc region or Fc molecule comprises an Fc domain with an L234A and/or L235A mutation, wherein 234 and 235 are the positions using EU numbering and corresponds to the positions as noted in Tables 3-5. The Fc domain can comprise an L234A mutation, an L235A mutation, a charged pair mutation, a hinge deletion, or any combination thereof. In some embodiments, the Fc domain comprises both an L234A mutation and an L235A mutation. In some embodiments, the Fc domain comprises a hinge deletion, an L234A mutation, an L235A mutation, and a charged pair mutation, such as FcΔ10(+, L234A/L235A), FcΔ10(-, L234A/L235A), FcΔ16(+, L234A/L235A), or FcΔ16(-, L234A/L235A). In some embodiments, the Fc domain comprises an optional C-terminal lysine, e.g., FcΔ10(+,K,L234A/L235A), FcΔ10(-,K,L234A/L235A), FcΔ16(+,K,L234A/L235A), or FcΔ16(-,K,L234A/L235A).

In some embodiments, the Fc region or Fc molecule comprises an Fc domain with a "cysteine clamp." A cysteine clamp mutation involves the introduction of a cysteine into the Fc domain at a specific location through mutation so that when incubated with another Fc domain that also has a cysteine introduced at a specific location through mutation, a disulfide bond (cysteine clamp) may be formed between the two Fc domains (e.g., between an FcΔ16 (+) domain having a "cysteine clamp" mutation and an FcΔ16(-) domain having a "cysteine clamp" mutation). The cysteine can be introduced into the CH3 domain of an Fc domain. In some embodiments, an Fc domain may contain one or more such cysteine clamp mutations. In one embodiment, a cysteine clamp is provided by introducing a serine to cysteine mutation (S354C, wherein 354 is the position using EU numbering, and corresponds to the position as noted in Tables 3-5) into a first Fc domain and a tyrosine to cysteine mutation (Y349C, wherein 349 is the position using EU numbering, and corresponds to the position as noted in Tables 3-5) into a second Fc domain. In one embodiment, a GDF15 molecule comprises an Fc region comprising an Fc domain with a cysteine clamp, a negatively charged pair mutation and a sixteen-amino acid hinge deletion (e.g., GDF15- FcΔ16(-,CC)), and an Fc molecule comprising an Fc domain comprising a cysteine clamp, a positively charged pair mutation and a sixteen-amino acid hinge deletion, and an optional C-terminal lysine (e.g., FcΔ16(+,K,CC)). The cysteine clamp may augment the heterodimerization of the GDF-Fc molecule with the Fc molecule.

Examples of Fc regions that can be used in a GDF15 molecule are shown in Table 3.

**Table 3 - Fc Regions**

| **SEQ ID NO:** | **Designation** | **Sequence** |
|---|---|---|
| 26 | FcΔ10(-) | APELLGGPSVFLFPPKPKDTLMISRTPEVTCVVV DVSHEDPEVKFNWYVDGVEVHNAKTKPREEQ YNSTYRVVSVLTVLHQDWLNGKEYKCKVSNK ALPAPIEKTISKAKGQPREPQVYTLPPSREEMTK NQVSLTCLVKGFYPSDIAVEWESNGQPENNYDT |
| | | TPPVLDSDGSFFLYSDLTVDKSRWQQGNVFSCS VMHEALHNHYTQKSLSLSPG *Underlined and bolded residues are K392D and K409D mutations.* |
| 27 | FcΔ10(+) | APELLGGPSVFLFPPKPKDTLMISRTPEVTCVVV DVSHEDPEVKFNWYVDGVEVHNAKTKPREEQ YNSTYRVVSVLTVLHQDWLNGKEYKCKVSNK ALPAPIEKTISKAKGQPREPQVYTLPPSRKEMTK NQVSLTCLVKGFYPSDIAVEWESNGQPENNYKT TPPVLKSDGSFFLYSKLTVDKSRWQQGNVFSCS VMHEALHNHYTQKSLSLSPG *Underlined and bolded residues are E356K and D399K mutations.* |
| 28 | FcΔ10(-,CC) | APELLGGPSVFLFPPKPKDTLMISRTPEVTCVVV DVSHEDPEVKFNWYVDGVEVHNAKTKPREEQ YNSTYRVVSVLTVLHQDWLNGKEYKCKVSNK ALPAPIEKTISKAKGQPREPQVCTLPPSREEMTK NQVSLTCLVKGFYPSDIAVEWESNGQPENNYDT TPPVLDSDGSFFLYSDLTVDKSRWQQGNVFSCS VMHEALHNHYTQKSLSLSPG *Underlined and italicized residue is Y349C mutation; underlined and bolded residues are K392D and K409D mutations.* |
| 29 | FcΔ16(-,CC) | GPSVFLFPPKPKDTLMISRTPEVTCVVVDVSHED PEVKFNWYVDGVEVHNAKTKPREEQYNSTYRV VSVLTVLHQDWLNGKEYKCKVSNKALPAPIEK TISKAKGQPREPQVCTLPPSREEMTKNQVSLTCL VKGFYPSDIAVEWESNGQPENNYDTTPPVLDSD GSFFLYSDLTVDKSRWQQGNVFSCSVMHEALH NHYTQKSLSLSPG *Underlined and italicized residue is Y349C mutation; underlined and bolded residues are K392D and K409D mutations.* |
| 30 | FcΔ16(-) | GPSVFLFPPKPKDTLMISRTPEVTCVVVDVSHED PEVKFNWYVDGVEVHNAKTKPREEQYNSTYRV VSVLTVLHQDWLNGKEYKCKVSNKALPAPIEK TISKAKGQPREPQVYTLPPSREEMTKNQVSLTCL VKGFYPSDIAVEWESNGQPENNYDTTPPVLDSD GSFFLYSDLTVDKSRWQQGNVFSCSVMHEALH NHYTQKSLSLSPG *Underlined and bolded residues are K392D and K409D mutations.* |
| 31 | FcΔ10(-,L234A/L235A) | APEAAGGPSVFLFPPKPKDTLMISRTPEVTCVVV DVSHEDPEVKFNWYVDGVEVHNAKTKPREEQ YNSTYRVVSVLTVLHQDWLNGKEYKCKVSNK ALPAPIEKTISKAKGQPREPQVYTLPPSREEMTK NQVSLTCLVKGFYPSDIAVEWESNGQPENNYDT TPPVLDSDGSFFLYSDLTVDKSRWQQGNVFSCS VMHEALHNHYTQKSLSLSPG *Underlined and italicized residues are L234A and L235A mutations; underlined and bolded residues are K392D and K409D mutations.* |

Examples of Fc molecules are shown in Table 4, in which the C-terminal lysine is optional.

**Table 4 - Fc Molecules**

| **SEQ ID NO:** | **Designation** | **Sequence** |
|---|---|---|
| 32 | FcΔ10(+,K) | APELLGGPSVFLFPPKPKDTLMISRTPEVTCV VVDVSHEDPEVKFNWYVDGVEVHNAKTKP REEQYNSTYRVVSVLTVLHQDWLNGKEYKC KVSNKALPAPIEKTISKAKGQPREPQVYTLPP SRKEMTKNQVSLTCLVKGFYPSDIAVEWESN GQPENNYKTTPPVLKSDGSFFLYSKLTVDKS RWQQGNVFSCSVMHEALHNHYTQKSLSLSP GK *Underlined and bolded residues are E356K and D399K mutations.* |
| 33 | FcΔ10(-,K) | APELLGGPSVFLFPPKPKDTLMISRTPEVTCV VVDVSHEDPEVKFNWYVDGVEVHNAKTKP REEQYNSTYRVVSVLTVLHQDWLNGKEYKC KVSNKALPAPIEKTISKAKGQPREPQVYTLPP SREEMTKNQVSLTCLVKGFYPSDIAVEWESN GQPENNYDTTPPVLDSDGSFFLYSDLTVDKS RWQQGNVFSCSVMHEALHNHYTQKSLSLSP GK *Underlined and bolded residues are K392D and K409D mutations.* |
| 34 | FcΔ10(+,K,CC) | APELLGGPSVFLFPPKPKDTLMISRTPEVTCV VVDVSHEDPEVKFNWYVDGVEVHNAKTKP REEQYNSTYRVVSVLTVLHQDWLNGKEYKC |
| | | KVSNKALPAPIEKTISKAKGQPREPQVYTLPP CRKEMTKNQVSLTCLVKGFYPSDIAVEWESN GQPENNYKTTPPVLKSDGSFFLYSKLTVDKS RWQQGNVFSCSVMHEALHNHYTQKSLSLSP GK *Underlined and italicized residue is S354C mutation; underlined and bolded residues are E356K and D399K mutations.* |
| 35 | FcΔ16(+,K,CC) | GPSVFLFPPKPKDTLMISRTPEVTCVVVDVSH EDPEVKFNWYVDGVEVHNAKTKPREEQYNS TYRVVSVLTVLHQDWLNGKEYKCKVSNKAL PAPIEKTISKAKGQPREPQVYTLPPCRKEMTK NQVSLTCLVKGFYPSDIAVEWESNGQPENNY KTTPPVLKSDGSFFLYSKLTVDKSRWQQGNV FSCSVMHEALHNHYTQKSLSLSPGK *Underlined and italicized residue is S354C mutation; underlined and bolded residues are E356K and D399K mutations.* |
| 36 | FcΔ16(+,K) | GPSVFLFPPKPKDTLMISRTPEVTCVVVDVSH EDPEVKFNWYVDGVEVHNAKTKPREEQYNS TYRVVSVLTVLHQDWLNGKEYKCKVSNKAL PAPIEKTISKAKGQPREPQVYTLPPSRKEMTK NQVSLTCLVKGFYPSDIAVEWESNGQPENNY KTTPPVLKSDGSFFLYSKLTVDKSRWQQGNV FSCSVMHEALHNHYTQKSLSLSPGK *Underlined and bolded residues are E356K and D399K mutations.* |
| 37 | FcΔ10(+,K,L234A/L235A) | APEAAGGPSVFLFPPKPKDTLMISRTPEVTCV VVDVSHEDPEVKFNWYVDGVEVHNAKTKP REEQYNSTYRVVSVLTVLHQDWLNGKEYKC KVSNKALPAPIEKTISKAKGQPREPQVYTLPP SRKEMTKNQVSLTCLVKGFYPSDIAVEWESN GQPENNYKTTPPVLKSDGSFFLYSKLTVDKS RWQQGNVFSCSVMHEALHNHYTQKSLSLSP GK *Underlined and italicized residues are L234A and L235A mutations; underlined and bolded residues are E356K and D399K mutations.* |

The Fc molecules can be used to dimerize with a molecule comprising a complementary Fc domain. For example, an Fc molecule of FcΔ10(+,K) can dimerize with a molecule comprising an Fc region comprising a ten-amino acid hinge deletion and a negatively charged pair mutation such as FcΔ10(-) (e.g., a GDF15 molecule comprising an Fc region of FcΔ10(-)). An Fc molecule of FcΔ10(-,K)can dimerize with a molecule comprising an Fc region comprising a ten-amino acid hinge deletion and a negatively charged pair mutation such as FcΔ10(+) (e.g., a GDF15 molecule comprising an Fc region of FcΔ10(+)).

An Fc molecule of FcΔ10(+,K,CC) can dimerize with a molecule comprising an Fc region comprising a ten-amino acid hinge deletion and a negatively charged pair mutation such as FcΔ10(-,CC) (e.g., a GDF15 molecule comprising an Fc region of FcΔ10(-, CC)). An Fc molecule of FcΔ16(+,K,CC) can dimerize with a molecule comprising an Fc region comprising a ten-amino acid hinge deletion and a negatively charged pair mutation such as FcΔ16(-, CC) (e.g., a GDF15 molecule comprising an Fc region of FcΔ16(-, CC)). An Fc molecule of FcΔ16(+,K) can dimerize with a molecule comprising an Fc region comprising a ten-amino acid hinge deletion and a negatively charged pair mutation such as FcΔ16(-) (e.g., a GDF15 molecule comprising an Fc region of FcΔ16(+)). An Fc molecule of FcΔ10(+,K,L234A/L235A) can dimerize with a molecule comprising an Fc region comprising a ten-amino acid hinge deletion and a negatively charged pair mutation such as FcΔ10(-,L234A/L235A) (e.g., a GDF15 molecule comprising an Fc region of FcΔ10(-, L234A/L235A)).

Examples of GDF15 molecules that are GDF15-Fc fusion proteins are shown in Table 5.

**Table 5 - GDF15 Molecules: SEQ ID NOs: 50, 52, 54 and 57 are embodiments of the invention.**

| **GDF15-Fc Fusion Protein** | | | **GDF15-Fc Fusion Protein Components SEQ ID NOs** | | |
|---|---|---|---|---|---|
| **SEQ ID NO.** | **Designation** | **Sequence** | **Fc Region** | **Linker** | **GDF15 Region** |
| 38 | scFc-GDF15 | GGGERKSSVECPPCPAPP VAGPSVFLFPPKPKDTLMI SRTPEVTCVVVDVSHEDP EVQFNWYVDGVEVHNA KTKPREEQFNSTFRVVSV LTVVHQDWLNGKEYKCK VSNKGLPAPIEKTISKTKG QPREPQVYTLPPSREEMT KNQVSLTCLVKGFYPSDI AVEWESNGQPENNYKTT PPMLDSDGSFFLYSKLTV DKSRWQQGNVFSCSVMH | --- | --- | --- |
| | | EALHNHYTQKSLSLSPGG GGGSGGGGSGGGGSGGG GSGGGGSGGGGSGGGGS GGGGSERKSSVECPPCPA PPVAGPSVFLFPPKPKDTL MISRTPEVTCVVVDVSHE DPEVQFNWYVDGVEVHN AKTKPREEQFNSTFRVVS VLTVVHQDWLNGKEYKC KVSNKGLPAPIEKTISKTK GQPREPQVYTLPPSREEM TKNQVSLTCLVKGFYPSD IAVEWESNGQPENNYKTT PPMLDSDGSFFLYSKLTV DKSRWQQGNVFSCSVMH EALHNHYTQKSLSLSPGS GGGGSGGGGSGGGGSGG GGSARNGDHCPLGPGRC CRLHTVRASLEDLGWAD WVLSPREVQVTMCIGACP SQFRAANMHAQIKTSLHR LKPDTVPAPCCVPASYNP MVLIQKTDTGVSLQTYDD LLAKDCHCI | | | |
| 39 | FcΔ10(-)-(G4S)4-GDF15 | APELLGGPSVFLFPPKPKD TLMISRTPEVTCVVVDVS HEDPEVKFNWYVDGVEV HNAKTKPREEQYNSTYR VVSVLTVLHQDWLNGKE YKCKVSNKALPAPIEKTIS KAKGQPREPQVYTLPPSR EEMTKNQVSLTCLVKGF YPSDIAVEWESNGQPENN YDTTPPVLDSDGSFFLYS DLTVDKSRWQQGNVFSC SVMHEALHNHYTQKSLS LSPGGGGGSGGGGSGGG GSGGGGSARNGDHCPLG PGRCCRLHTVRASLEDLG WADWVLSPREVQVTMCI GACPSQFRAANMHAQIKT SLHRLKPDTVPAPCCVPA SYNPMVLIQKTDTGVSLQ TYDDLLAKDCHCI | 26 | 21 | 6 |
| | | *Underlined and bolded residues are K392D and K409D mutations.* | | | |
| 40 | FcΔ10(+)-(G4)-GDF15 | APELLGGPSVFLFPPKPKD TLMISRTPEVTCVVVDVS HEDPEVKFNWYVDGVEV HNAKTKPREEQYNSTYR VVSVLTVLHQDWLNGKE YKCKVSNKALPAPIEKTIS KAKGQPREPQVYTLPPSR KEMTKNQVSLTCLVKGF YPSDIAVEWESNGQPENN YKTTPPVLKSDGSFFLYS KLTVDKSRWQQGNVFSC SVMHEALHNHYTQKSLS LSPGGGGGARNGDHCPL GPGRCCRLHTVRASLEDL GWADWVLSPREVQVTM CIGACPSQFRAANMHAQI KTSLHRLKPDTVPAPCCV PASYNPMVLIQKTDTGVS LQTYDDLLAKDCHCI | 27 | 23 | 6 |
| | | *Underlined and and bolded residues are E356K and D399K mutations.* | | | |
| 41 | FcΔ10(-)-GDF15(Δ3) | APELLGGPSVFLFPPKPKD TLMISRTPEVTCVVVDVS HEDPEVKFNWYVDGVEV HNAKTKPREEQYNSTYR VVSVLTVLHQDWLNGKE YKCKVSNKALPAPIEKTIS KAKGQPREPQVYTLPPSR EEMTKNQVSLTCLVKGF YPSDIAVEWESNGQPENN YDTTPPVLDSDGSFFLYS DLTVDKSRWQQGNVFSC SVMHEALHNHYTQKSLS LSPGGDHCPLGPGRCCRL HTVRASLEDLGWADWVL SPREVQVTMCIGACPSQF RAANMHAQIKTSLHRLKP DTVPAPCCVPASYNPMVL IQKTDTGVSLQTYDDLLA KDCHCI | 26 | - | 13 |
| | | *Underlined and bolded residues are K392D and K409D mutations.* | | | |
| 42 | FcΔ10(-)-GDF15(N3D) | APELLGGPSVFLFPPKPKD TLMISRTPEVTCVVVDVS HEDPEVKFNWYVDGVEV HNAKTKPREEQYNSTYR VVSVLTVLHQDWLNGKE YKCKVSNKALPAPIEKTIS KAKGQPREPQVYTLPPSR EEMTKNQVSLTCLVKGF YPSDIAVEWESNGQPENN YDTTPPVLDSDGSFFLYS DLTVDKSRWQQGNVFSC SVMHEALHNHYTQKSLS LSPGARDGDHCPLGPGRC CRLHTVRASLEDLGWAD WVLSPREVQVTMCIGACP SQFRAANMHAQIKTSLHR LKPDTVPAPCCVPASYNP MVLIQKTDTGVSLQTYDD LLAKDCHCI | 26 | - | 15 |
| | | *Underlined and bolded residues are K392D and K409D mutations.* | | | |
| 43 | FcΔ10(-,CC)-GDF15(Δ3) | APELLGGPSVFLFPPKPKD TLMISRTPEVTCVVVDVS HEDPEVKFNWYVDGVEV HNAKTKPREEQYNSTYR VVSVLTVLHQDWLNGKE YKCKVSNKALPAPIEKTIS KAKGQPREPQVCTLPPSR EEMTKNQVSLTCLVKGF YPSDIAVEWESNGQPENN YDTTPPVLDSDGSFFLYS DLTVDKSRWQQGNVFSC SVMHEALHNHYTQKSLS LSPGGDHCPLGPGRCCRL HTVRASLEDLGWADWVL SPREVQVTMCIGACPSQF RAANMHAQIKTSLHRLKP DTVPAPCCVPASYNPMVL IQKTDTGVSLQTYDDLLA KDCHCI | 28 | - | 13 |
| | | *Underlined and italicized residue is Y349C mutation; underlined and bolded residues are K392D and K409D mutations.* | | | |
| 44 | FcΔ10(-,CC)-GDF15(N3D) | APELLGGPSVFLFPPKPKD TLMISRTPEVTCVVVDVS HEDPEVKFNWYVDGVEV HNAKTKPREEQYNSTYR VVSVLTVLHQDWLNGKE YKCKVSNKALPAPIEKTIS KAKGQPREPQVCTLPPSR EEMTKNQVSLTCLVKGF YPSDIAVEWESNGQPENN YDTTPPVLDSDGSFFLYS DLTVDKSRWQQGNVFSC SVMHEALHNHYTQKSLS LSPGARDGDHCPLGPGRC CRLHTVRASLEDLGWAD WVLSPREVQVTMCIGACP SQFRAANMHAQIKTSLHR LKPDTVPAPCCVPASYNP MVLIQKTDTGVSLQTYDD LLAKDCHCI | 28 | - | 15 |
| | | *Underlined and italicized residue is Y349C mutation; underlined and bolded residues are K392D and K409D mutations.* | | | |
| 45 | FcΔ16(-,CC)-GDF15(Δ3/D5 E) | GPSVFLFPPKPKDTLMISRTP EVTCVVVDVSHEDPEVKFN WYVDGVEVHNAKTKPREEQ YNSTYRVVSVLTVLHQDWL NGKEYKCKVSNKALPAPIEK TISKAKGQPREPQVCTLPPSR EEMTKNQVSLTCLVKGFYPS DIAVEWESNGQPENNYDTTP PVLDSDGSFFLYSDLTVDKS RWQQGNVFSCSVMHEALHN HYTQKSLSLSPGGEHCPLGP GRCCRLHTVRASLEDLGWA DWVLSPREVQVTMCIGACPS QFRAANMHAQIKTSLHRLKP DTVPAPCCVPASYNPMVLIQ KTDTGVSLQTYDDLLAKDC HCI | 29 | - | 17 |
| | | *Underlined and italicized residue is Y349C mutation; underlined and bolded residues are K392D and K409D mutations.* | | | |
| 46 | FcΔ16(-,CC)-GDF15(N3Q/ D5E) | GPSVFLFPPKPKDTLMISRTP EVTCVVVDVSHEDPEVKFN WYVDGVEVHNAKTKPREEQ YNSTYRVVSVLTVLHQDWL NGKEYKCKVSNKALPAPIEK TISKAKGQPREPQVCTLPPSR EEMTKNQVSLTCLVKGFYPS DIAVEWESNGQPENNYDTTP PVLDSDGSFFLYSDLTVDKS RWQQGNVFSCSVMHEALHN HYTQKSLSLSPGARQGEHCP LGPGRCCRLHTVRASLEDLG WADWVLSPREVQVTMCIGA CPSQFRAANMHAQIKTSLHR LKPDTVPAPCCVPASYNPMV LIQKTDTGVSLQTYDDLLAK DCHCI | 29 | - | 18 |
| | | *Underlined and italicized residue is Y349C mutation; underlined and bolded residues are K392D and K409D mutations.* | | | |
| 47 | FcΔ16(-)-GDF15(N3Q/ D5E) | GPSVFLFPPKPKDTLMISR TPEVTCVVVDVSHEDPEV KFNWYVDGVEVHNAKT KPREEQYNSTYRVVSVLT VLHQDWLNGKEYKCKVS NKALPAPIEKTISKAKGQP REPQVYTLPPSREEMTKN QVSLTCLVKGFYPSDIAV EWESNGQPENNYDTTPPV LDSDGSFFLYSDLTVDKS RWQQGNVFSCSVMHEAL HNHYTQKSLSLSPGARQG EHCPLGPGRCCRLHTVRA SLEDLGWADWVLSPREV QVTMCIGACPSQFRAAN MHAQIKTSLHRLKPDTVP APCCVPASYNPMVLIQKT DTGVSLQTYDDLLAKDC HCI | 30 | - | 18 |
| | | *Underlined and bolded residues are K392D and K409D mutations.* | | | |
| 48 | FcΔ16(-)-(G4Q)4-GDF15 | GPSVFLFPPKPKDTLMISR TPEVTCVVVDVSHEDPEV KFNWYVDGVEVHNAKT KPREEQYNSTYRVVSVLT VLHQDWLNGKEYKCKVS NKALPAPIEKTISKAKGQP REPQVYTLPPSREEMTKN QVSLTCLVKGFYPSDIAV EWESNGQPENNYDTTPPV LDSDGSFFLYSDLTVDKS RWQQGNVFSCSVMHEAL HNHYTQKSLSLSPGGGGG QGGGGQGGGGQGGGGQ ARNGDHCPLGPGRCCRL HTVRASLEDLGWADWVL SPREVQVTMCIGACPSQF RAANMHAQIKTSLHRLKP DTVPAPCCVPASYNPMVL IQKTDTGVSLQTYDDLLA KDCHCI | 30 | 25 | 6 |
| | | *Underlined and bolded residues are K392D and K409D mutations.* | | | |
| 49 | FcΔ16(-)-(G4Q)4-GDF15(N3Q) | GPSVFLFPPKPKDTLMISR TPEVTCVVVDVSHEDPEV KFNWYVDGVEVHNAKT KPREEQYNSTYRVVSVLT VLHQDWLNGKEYKCKVS NKALPAPIEKTISKAKGQP REPQVYTLPPSREEMTKN QVSLTCLVKGFYPSDIAV EWESNGQPENNYDTTPPV LDSDGSFFLYSDLTVDKS RWQQGNVFSCSVMHEAL HNHYTQKSLSLSPGGGGG QGGGGQGGGGQGGGGQ ARQGDHCPLGPGRCCRL HTVRASLEDLGWADWVL SPREVQVTMCIGACPSQF RAANMHAQIKTSLHRLKP DTVPAPCCVPASYNPMVL | 30 | 25 | 14 |
| | | IQKTDTGVSLQTYDDLLA KDCHCI | | | |
| | | *Underlined and bolded residues are K392D and K409D mutations.* | | | |
| 50 | FcΔ16(-)-(G4Q)4-GDF15(N3Q/ D5E) | GPSVFLFPPKPKDTLMISRTP EVTCVVVDVSHEDPEVKFN WYVDGVEVHNAKTKPREEQ YNSTYRVVSVLTVLHQDWL NGKEYKCKVSNKALPAPIEK TISKAKGQPREPQVYTLPPSR EEMTKNQVSLTCLVKGFYPS DIAVEWESNGQPENNYDTTP PVLDSDGSFFLYSDLTVDKS RWQQGNVFSCSVMHEALHN HYTQKSLSLSPGGGGGQGG GGQGGGGQGGGGQARQGE HCPLGPGRCCRLHTVRASLE DLGWADWVLSPREVQVTM CIGACPSQFRAANMHAQIKT SLHRLKPDTVPAPCCVPASY NPMVLIQKTDTGVSLQTYD DLLAKDCHCI | 30 | 25 | 18 |
| | | *Underlined and bolded residues are K392D and K409D mutations.* | | | |
| 51 | FcΔ16(-)-(G4S)2-GDF15(N3Q) | GPSVFLFPPKPKDTLMISR TPEVTCVVVDVSHEDPEV KFNWYVDGVEVHNAKT KPREEQYNSTYRVVSVLT VLHQDWLNGKEYKCKVS NKALPAPIEKTISKAKGQP REPQVYTLPPSREEMTKN QVSLTCLVKGFYPSDIAV EWESNGQPENNYDTTPPV LDSDGSFFLYSDLTVDKS RWQQGNVFSCSVMHEAL HNHYTQKSLSLSPGGGGG SGGGGSARQGDHCPLGP GRCCRLHTVRASLEDLG WADWVLSPREVQVTMCI GACPSQFRAANMHAQIKT SLHRLKPDTVPAPCCVPA SYNPMVLIQKTDTGVSLQ TYDDLLAKDCHCI | 30 | 20 | 14 |
| | | *Underlined and bolded residues are K392D and K409D mutations.* | | | |
| 52 | FcΔ16(-)-(G4S)2-GDF15(N3Q/ D5E) | GPSVFLFPPKPKDTLMISR TPEVTCVVVDVSHEDPEV KFNWYVDGVEVHNAKT KPREEQYNSTYRVVSVLT VLHQDWLNGKEYKCKVS NKALPAPIEKTISKAKGQP REPQVYTLPPSREEMTKN QVSLTCLVKGFYPSDIAV EWESNGQPENNYDTTPPV LDSDGSFFLYSDLTVDKS RWQQGNVFSCSVMHEAL HNHYTQKSLSLSPGGGGG SGGGGSARQGEHCPLGPG RCCRLHTVRASLEDLGW ADWVLSPREVQVTMCIG ACPSQFRAANMHAQIKTS LHRLKPDTVPAPCCVPAS YNPMVLIQKTDTGVSLQT YDDLLAKDCHCI | 30 | 20 | 18 |
| | | *Underlined and bolded residues are K392D and K409D mutations.* | | | |
| 53 | FcΔ16(-)-G4S-GDF15(N3Q) | GPSVFLFPPKPKDTLMISR TPEVTCVVVDVSHEDPEV KFNWYVDGVEVHNAKT KPREEQYNSTYRVVSVLT VLHQDWLNGKEYKCKVS NKALPAPIEKTISKAKGQP REPQVYTLPPSREEMTKN QVSLTCLVKGFYPSDIAV EWESNGQPENNYDTTPPV LDSDGSFFLYSDLTVDKS RWQQGNVFSCSVMHEAL HNHYTQKSLSLSPGGGGG SARQGDHCPLGPGRCCRL HTVRASLEDLGWADWVL SPREVQVTMCIGACPSQF RAANMHAQIKTSLHRLKP DTVPAPCCVPASYNPMVL IQKTDTGVSLQTYDDLLA KDCHCI | 30 | 19 | 14 |
| | | *Underlined and bolded residues are K392D and K409D mutations.* | | | |
| 54 | FcΔ16(-)-G4S-GDF15(N3Q/ D5E) | GPSVFLFPPKPKDTLMISR TPEVTCVVVDVSHEDPEV KFNWYVDGVEVHNAKT KPREEQYNSTYRVVSVLT VLHQDWLNGKEYKCKVS NKALPAPIEKTISKAKGQP REPQVYTLPPSREEMTKN QVSLTCLVKGFYPSDIAV EWESNGQPENNYDTTPPV LDSDGSFFLYSDLTVDKS RWQQGNVFSCSVMHEAL HNHYTQKSLSLSPGGGGG SARQGEHCPLGPGRCCRL HTVRASLEDLGWADWVL SPREVQVTMCIGACPSQF RAANMHAQIKTSLHRLKP DTVPAPCCVPASYNPMVL IQKTDTGVSLQTYDDLLA KDCHCI | 30 | 19 | 18 |
| | | *Underlined and bolded residues are K392D and K409D mutations.* | | | |
| 55 | FcΔ16(-)-GDF15(N3Q) | GPSVFLFPPKPKDTLMISR TPEVTCVVVDVSHEDPEV KFNWYVDGVEVHNAKT KPREEQYNSTYRVVSVLT VLHQDWLNGKEYKCKVS NKALPAPIEKTISKAKGQP REPQVYTLPPSREEMTKN QVSLTCLVKGFYPSDIAV EWESNGQPENNYDTTPPV LDSDGSFFLYSDLTVDKS RWQQGNVFSCSVMHEAL HNHYTQKSLSLSPGARQG DHCPLGPGRCCRLHTVRA SLEDLGWADWVLSPREV QVTMCIGACPSQFRAAN MHAQIKTSLHRLKPDTVP APCCVPASYNPMVLIQKT DTGVSLQTYDDLLAKDC HCI | 30 | - | 14 |
| | | *Underlined and bolded residues are K392D and K409D mutations.* | | | |
| 56 | FcΔ10(-,L234A/L235 A)-(G4Q)4-GDF15(N3Q) | APEAAGGPSVFLFPPKPKD TLMISRTPEVTCVVVDVS HEDPEVKFNWYVDGVEV HNAKTKPREEQYNSTYR VVSVLTVLHQDWLNGKE YKCKVSNKALPAPIEKTIS KAKGQPREPQVYTLPPSR EEMTKNQVSLTCLVKGF YPSDIAVEWESNGQPENN YDTTPPVLDSDGSFFLYS DLTVDKSRWQQGNVFSC SVMHEALHNHYTQKSLS LSPGGGGGQGGGGQGGG GQGGGGQARQGDHCPLG PGRCCRLHTVRASLEDLG WADWVLSPREVQVTMCI GACPSQFRAANMHAQIKT SLHRLKPDTVPAPCCVPA SYNPMVLIQKTDTGVSLQ TYDDLLAKDCHCI | 31 | 25 | 14 |
| | | *Underlined and italicized residues are L234A and L235A mutations; underlined and bolded residues are K392D and K409D mutations.* | | | |
| 57 | FcΔ10(-,L234A/L235 A)-(G4Q)4-GDF15(N3Q/ D5E) | APEAAGGPSVFLFPPKPKD TLMISRTPEVTCVVVDVS HEDPEVKFNWYVDGVEV HNAKTKPREEQYNSTYR VVSVLTVLHQDWLNGKE YKCKVSNKALPAPIEKTIS KAKGQPREPQVYTLPPSR EEMTKNQVSLTCLVKGF YPSDIAVEWESNGQPENN YDTTPPVLDSDGSFFLYS DLTVDKSRWQQGNVFSC SVMHEALHNHYTQKSLS LSPGGGGGQGGGGQGGG GQGGGGQARQGEHCPLG PGRCCRLHTVRASLEDLG WADWVLSPREVQVTMCI | 31 | 25 | 18 |
| | | GACPSQFRAANMHAQIKT SLHRLKPDTVPAPCCVPA SYNPMVLIQKTDTGVSLQ TYDDLLAKDCHCI | | | |
| | | *Underlined and italicized residues are L234A and L235A mutations; underlined and bolded residues are K392D and K409D mutations.* | | | |

In some embodiments, the fusion protein is an scFc-GDF15 in which the GDF15 region is joined to two Fc regions. In some embodiments, the fusion protein comprises an amino acid sequence that has at least 85%, 90%, 95% or 99% sequence identity to SEQ ID NO: 38. In some embodiments, the fusion protein comprises an amino acid sequence of SEQ ID NO: 38. In calculating percent sequence identity, the sequences being compared are aligned in a way that gives the largest match between the sequences. A computer program that can be used to determine percent identity is the GCG program package, which includes GAP (Devereux et al., (1984) Nucl. Acid Res. 12:387; Genetics Computer Group, University of Wisconsin, Madison, Wis.). The computer algorithm GAP can be used to align the two polypeptides or polynucleotides for which the percent sequence identity is to be determined. The sequences are aligned for optimal matching of their respective amino acid or nucleotide (the "matched span", as determined by the algorithm). A gap opening penalty (which is calculated as 3x the average diagonal, wherein the "average diagonal" is the average of the diagonal of the comparison matrix being used; the "diagonal" is the score or number assigned to each perfect amino acid match by the particular comparison matrix) and a gap extension penalty (which is usually 1/10 times the gap opening penalty), as well as a comparison matrix such as PAM 250 or BLOSUM 62 are used in conjunction with the algorithm. In certain embodiments, a standard comparison matrix (see, Dayhoff et al., (1978) Atlas of Protein Sequence and Structure 5:345-352 for the PAM 250 comparison matrix; Henikoff et al., (1992) Proc. Natl. Acad. Sci. U.S.A. 9:10915-10919 for the BLOSUM 62 comparison matrix) is also used by the algorithm. Parameters that can be used for determining percent identity using the GAP program are the following:
Algorithm: Needleman et al., 1970, J. Mol. Biol. 48:443-453;
Comparison matrix: BLOSUM 62 from Henikoff et al., 1992, supra;
Gap Penalty: 12 (but with no penalty for end gaps)
Gap Length Penalty: 4
Threshold of Similarity: 0
Certain alignment schemes for aligning two amino acid sequences can result in matching of only a short region of the two sequences, and this small aligned region can have very high sequence identity even though there is no significant relationship between the two full-length sequences. Accordingly, the selected alignment method (e.g., the GAP program) can be adjusted if so desired to result in an alignment that spans at least 50 contiguous amino acids of the target polypeptide.

In some embodiments, the GDF15 molecule is FcΔ16(-)-(G4Q)4-GDF15(N3Q/D5E), FcΔ16(-)-(G4S)2- GDF15(N3Q/D5E), FcΔ16(-)-G4S-GDF15(N3Q/D5E), or FcΔ10(-,L234A/L235A)-(G4Q)4-GDF15(N3Q/D5E).

In some embodiments, the GDF15 molecule comprises the amino acid sequence of SEQ ID NO: 50, 52, 54, or 57. In some embodiments, the GDF15 molecule comprises an amino acid sequence that has 80-99%, 85%-99%, 90-99%, or 95-99% sequence identity to SEQ ID NO: 50, 52, 54, or 57. In some embodiments, the GDF15 molecule comprises an amino acid sequence that has at least 85% sequence identity to SEQ ID NO: 50, 52, 54, or 57. In some embodiments, the GDF15 molecule comprises an amino acid sequence that has at least 90% sequence identity to SEQ ID NO: 50, 52, 54, or 57. In some embodiments, the GDF15 molecule comprises an amino acid sequence that has at least 95% sequence identity to SEQ ID NO: 50, 52, 54, or 57. In some embodiments, the GDF15 molecule comprises an amino acid sequence that has at least 99% sequence identity to SEQ ID NO: 50, 52, 54, or 57.

In some embodiments, the GDF15 molecule is a FcΔ16(-)-(G4Q)4-GDF15(N3Q/D5E), FcΔ16(-)-(G4S)2- GDF15(N3Q/D5E), FcΔ16(-)-G4S-GDF15(N3Q/D5E), 4 or FcΔ10(-,L234A/L235A)-(G4Q)4-GDF15(N3Q/D5E) molecule. In some embodiments, the GDF15 molecule is a FcΔ16(-)-(G4Q)4-GDF15(N3Q/D5E), FcΔ16(-)-(G4S)2- GDF15(N3Q/D5E), FcΔ16(-)-G4S-GDF15(N3Q/D5E), or FcΔ10(-,L234A/L235A)-(G4Q)4-GDF15(N3Q/D5E) molecule that has 80-99%, 85%-99%, 90-99%, or 95-99% sequence identity to its Fc region and/or GDF15 region.

In some embodiments, the GDF15 molecule is a FcΔ16(-)-(G4Q)4-GDF15(N3Q/D5E), FcΔ16(-)-(G4S)2- GDF15(N3Q/D5E), FcΔ16(-)-G4S-GDF15(N3Q/D5E), F or FcΔ10(-,L234A/L235A)-(G4Q)4-GDF15(N3Q/D5E) molecule that has at least 85%, 90%, 95% or 99% sequence identity to its Fc region and/or GDF15 region. For example, a FcΔ10(-)-(G4S)4-GDF15 molecule with 80-99%, 85%-99%, 90-99%, or 95-99% sequence identity to its Fc region and/or GDF15 region, includes a GDF15 molecule with an Fc region that has a ten-amino acid deletion of the hinge region and a negatively charged pair mutation, and has 80-99%, 85%-99%, 90-99%, or 95-99% sequence identity to SEQ ID NO: 26 and/or a GDF15 region that has 80-99%, 85%-99%, 90-99%, or 95-99% sequence identity to SEQ ID NO: 6.

In one example, a FcΔ16(-)-(G4Q)4-GDF15(N3Q/D5E) molecule with 80-99%, 85%-99%, 90-99%, or 95-99% sequence identity to its Fc region and/or a GDF15 region, includes a GDF15 molecule with an Fc region that has a sixteen-amino acid deletion of the hinge region and a negatively charged pair mutation that has 80-99%, 85%-99%, 90-99%, or 95-99% sequence identity to SEQ ID NO: 30 and/or a GDF15 region that has 80-99%, 85%-99%, 90-99%, or 95-99% sequence identity to SEQ ID NO: 18. In another example, a FcΔ16(-)-(G4Q)4-GDF15(N3Q/D5E) molecule with at least 85%, 90%, 95% or 99% sequence identity to its Fc region and/or a GDF15 region, includes a GDF15 molecule with an Fc region that has a sixteen-amino acid deletion of the hinge region and a negatively charged pair mutation that has at least 85%, 90%, 95% or 99% sequence identity to SEQ ID NO: 30 and/or a GDF15 region that has at least 85%, 90%, 95% or 99% sequence identity to SEQ ID NO: 18.

In another example, a FcΔ10(-,L234A/L235A)-(G4Q)4-GDF15(N3Q/D5E) molecule with 80-99%, 85%-99%, 90-99%, or 95-99% sequence identity to its Fc region and/or a GDF15 region, includes a GDF15 molecule with an Fc region that has a ten-amino acid deletion of the hinge region, a negatively charged pair mutation and leucine to alanine mutations at positions 234 and 235 and has 80-99%, 85%-99%, 90-99%, or 95-99% sequence identity to SEQ ID NO: 31 and/or a GDF15 region that has 80-99%, 85%-99%, 90-99%, or 95-99% sequence identity to SEQ ID NO: 18. In yet another example, a FcΔ10(-,L234A/L235A)-(G4Q)4-GDF15(N3Q/D5E) molecule with at least 85%, 90%, 95% or 99% sequence identity to its Fc region and/or a GDF15 region, includes a GDF15 molecule with an Fc region that has a ten-amino acid deletion of the hinge region, a negatively charged pair mutation and leucine to alanine mutations at positions 234 and 235 and has at least 85%, 90%, 95% or 99% sequence identity to SEQ ID NO: 31 and/or a GDF15 region that has at least 85%, 90%, 95% or 99% sequence identity to SEQ ID NO: 18.

Also provided herein are dimers and tetramers comprising a GDF15 molecule provided herein. In one embodiment, the dimer comprises a GDF15-Fc fusion comprising the amino acid sequence of any one of SEQ ID NOs: 50, 52, 54 or 57. In some embodiments, a GDF15-Fc fusion comprising the amino acid sequence of SEQ ID NO: 50, 52, 54, or 57 dimerizes with an Fc molecule comprising the amino acid sequence of SEQ ID NO: 32, 33, 34, 35, 36, or 37 (in which the C-terminal lysine is optional), such as shown in Table 6. For example, in some embodiments, the dimer is FcΔ10(-,L234A/L235A)-(G4Q)4-GDF15(N3Q):FcΔ10(+,K,L234A/L235A).

**Table 6 - Dimers: SEQ ID Nos 50, 52, 54 and 57 are part of the claimed invention**

| **GDF15-Fc Fusion SEQ ID NO.** | **GDF15-Fc Fusion Designation** | **Fc Molecule SEQ ID NO.** | **Corresponding Fc Molecule Designation** |
|---|---|---|---|
| 39 | FcΔ10(-)-(G4S)4-GDF15 | 32 | FcΔ10(+,K) |
| 40 | FcΔ10(+)-(G4)-GDF15 | 33 | FcΔ10(-,K) |
| 41 | FcΔ10(-)-GDF15(Δ3) | 32 | FcΔ10(+,K) |
| 42 | FcΔ10(-)-GDF15(N3D) | 32 | FcΔ10(+,K) |
| 43 | FcΔ10(-,CC)-GDF15(Δ3) | 34 | FcΔ10(+,K,CC) |
| 44 | FcΔ10(-,CC)-GDF15(N3D) | 34 | FcΔ10(+,K,CC) |
| 45 | FcΔ16(-,CC)-GDF15(A3/D5E) | 35 | FcΔ16(+,K,CC) |
| 46 | FcΔ16(-,CC)-GDF15(N3Q/D5E) | 35 | FcΔ16(+,K,CC) |
| 47 | FcΔ16(-)-GDF15(N3Q/D5E) | 36 | FcΔ16(+,K) |
| 48 | FcΔ16(-)-(G4Q)4-GDF15 | 36 | FcΔ16(+,K) |
| 49 | FcΔ16(-)-(G4Q)4-GDF15(N3Q) | 36 | FcΔ16(+,K) |
| 50 | FcΔ16(-)-(G4Q)4-GDF15(N3Q/D5E) | 36 | FcΔ16(+,K) |
| 51 | FcΔ16(-)-(G4S)2-GDF15(N3Q) | 36 | FcΔ16(+,K) |
| 52 | FcΔ16(-)-(G4S)2-GDF15(N3Q/D5E) | 36 | FcΔ16(+,K) |
| 53 | FcΔ16(-)-G4S- GDF15(N3Q) | 36 | FcΔ16(+,K) |
| 54 | FcΔ16(-)-G4S-GDF15(N3Q/D5E) | 36 | FcΔ16(+,K) |
| 55 | FcΔ16(-)-GDF15(N3Q) | 36 | FcΔ16(+,K) |
| 56 | FcΔ10(-,L234A/L235A)-(G4Q)4-GDF15(N3Q) | 37 | FcΔ10(+,K,L234A/L235A) |
| 57 | FcΔ10(-,L234A/L235A)-(G4Q)4- GDF15(N3Q/D5E) | 37 | FcΔ10(+,K,L234A/L235A) |

In one embodiment, a GDF15-Fc fusion comprising the amino acid sequence of SEQ ID NO: 50 dimerizes with an Fc molecule comprising SEQ ID NO: 36 (C-terminal lysine optional). In another embodiment, a GDF15-Fc fusion comprising the amino acid sequence of SEQ ID NO: 52 dimerizes with an Fc molecule comprising SEQ ID NO: 36 (C-terminal lysine optional). In another embodiment, a GDF15-Fc fusion comprising the amino acid sequence of SEQ ID NO: 54 dimerizes with an Fc molecule comprising SEQ ID NO: 36 (C-terminal lysine optional). In another embodiment, a GDF15-Fc fusion comprising the amino acid sequence of SEQ ID NO: 57 dimerizes with an Fc molecule comprising SEQ ID NO: 37 (C-terminal lysine optional).

In some embodiments, the dimers form tetramers. For example, the dimers in Table 6 can form tetramers. In some embodiments, the tetramers are formed from the same dimers. In some embodiments, two dimers of FcΔ16(-)-(G4Q)4-GDF15(N3Q/D5E):FcΔ16(+,K); FcΔ16(-)-(G4S)2- GDF15(N3Q/D5E):FcΔ16(+,K); FcΔ16(-)-G4S-GDF15(N3Q/D5E): FcΔ16(+,Kor FcΔ10(-,L234A/L235A)-(G4Q)4-GDF15(N3Q/D5E):FcΔ10(+,K,L234A/L235A) form a tetramer, such as through the dimerization of the two GDF15 regions.

Also provided herein are host cells comprising the nucleic acids and vectors for producing the GDF15 and Fc molecules disclosed herein. In various embodiments, the vector or nucleic acid is integrated into the host cell genome, which in other embodiments the vector or nucleic acid is extra-chromosomal.

Recombinant cells, such as yeast, bacterial *(e.g., E. coli),* and mammalian cells *(e.g.,* immortalized mammalian cells) comprising such a nucleic acid, vector, or combinations of either or both thereof are provided. In various embodiments, cells comprising a non-integrated nucleic acid, such as a plasmid, cosmid, phagemid, or linear expression element, which comprises a sequence coding for expression of a GDF15 molecule and/or an Fc molecule. In some embodiments, the cell comprises a nucleic acid for producing a GDF15 molecule and another cell comprises a nucleic acid for producing an Fc molecule for dimerization with the GDF15 molecule (e.g., a vector for encoding a GDF15 molecule in one cell and a second vector for encoding an Fc molecule in a second cell). In other embodiments, a host cell comprises a nucleic acid for producing a GDF15 molecule and an Fc molecule (e.g., a vector that encodes both molecules). In another embodiment, a host cell comprises a nucleic acid for producing a GDF15 molecule and another nucleic acid for producing an Fc molecule (e.g., two separate vectors, one that encodes a GDF15 molecule and one that encodes an Fc molecule, in a single host cell).

A vector comprising a nucleic acid sequence encoding a GDF15 molecule and/or an Fc molecule can be introduced into a host cell by transformation or by transfection, such as by methods known in the art.

A nucleic acid encoding a GDF15 molecule can be positioned in and/or delivered to a host cell or host animal via a viral vector. A viral vector can comprise any number of viral polynucleotides, alone or in combination with one or more viral proteins, which facilitate delivery, replication, and/or expression of the nucleic acid of the invention in a desired host cell. The viral vector can be a polynucleotide comprising all or part of a viral genome, a viral protein/nucleic acid conjugate, a virus-like particle (VLP), or an intact virus particle comprising viral nucleic acids and a nucleic acid encoding a polypeptide comprising a GDF15 region. A viral particle viral vector can comprise a wild-type viral particle or a modified viral particle. The viral vector can be a vector which requires the presence of another vector or wild-type virus for replication and/or expression (e.g., a viral vector can be a helper-dependent virus), such as an adenoviral vector amplicon. Suitable viral vector particles in this respect, include, for example, adenoviral vector particles (including any virus of or derived from a virus of the adenoviridae), adeno-associated viral vector particles (AAV vector particles) or other parvoviruses and parvoviral vector particles, papillomaviral vector particles, flaviviral vectors, alphaviral vectors, herpes viral vectors, pox virus vectors, retroviral vectors, including lentiviral vectors.

A GDF15 molecule can be isolated using standard protein purification methods. A polypeptide comprising a GDF15 region can be isolated from a cell that has been engineered to express a polypeptide comprising a GDF15 region, for example a cell that does not naturally express native GDF15. Protein purification methods known in the art can be employed to isolate GDF15 molecules, as well as associated materials and reagents. Methods of purifying a GDF15 molecule are also provided in the Examples herein. Additional purification methods that may be useful for isolating GDF15 molecules can be found in references such as Bootcov MR, 1997, Proc. Natl. Acad. Sci. USA 94:11514-9, Fairlie WD, 2000, Gene 254: 67-76.

Pharmaceutical compositions comprising a GDF15 molecule (and optionally, an Fc molecule, such as a dimer or tetramer disclosed herein) are also provided. Such polypeptide pharmaceutical compositions can comprise a therapeutically effective amount of a GDF15 molecule in admixture with a pharmaceutically or physiologically acceptable formulation agent or carrier selected for suitability with the mode of administration. The pharmaceutically or physiologically acceptable formulation agent can be one or more formulation agents suitable for accomplishing or enhancing the delivery of a GDF15 molecule into the body of a human or non-human subject. Pharmaceutically acceptable substances such as wetting or emulsifying agents, preservatives or buffers, which enhance the shelf life or effectiveness of the GDF15 molecule can also act as, or form a component of, a formulation carrier. Acceptable pharmaceutically acceptable carriers are preferably nontoxic to recipients at the dosages and concentrations employed. The pharmaceutical composition can contain formulation agent(s) for modifying, maintaining, or preserving, for example, the pH, osmolarity, viscosity, clarity, color, isotonicity, odor, sterility, stability, rate of dissolution or release, adsorption, or penetration of the composition.

The effective amount of pharmaceutical composition comprising a GDF15 molecule which is to be employed therapeutically will depend, for example, upon the therapeutic context and objectives. One skilled in the art will appreciate that the appropriate dosage levels for treatment will thus vary depending, in part, upon the molecule delivered, the indication for which a GDF15 molecule is being used, the route of administration, and the size (body weight, body surface, or organ size) and condition (the age and general health) of the subject. The frequency of dosing will depend upon the pharmacokinetic parameters of the GDF15 molecule in the formulation being used.

The route of administration of the pharmaceutical composition can be orally; through injection by intravenous, intraperitoneal, intracerebral (intraparenchymal), intracerebroventricular, intramuscular, intraocular, intraarterial, intraportal, or intralesional routes; by sustained release systems (which may also be injected); or by implantation devices. Where desired, the compositions can be administered by bolus injection or continuously by infusion, or by an implantation device. The composition can also be administered locally via implantation of a membrane, sponge, or other appropriate material onto which the desired molecule has been absorbed or encapsulated. Where an implantation device is used, the device can be implanted into any suitable tissue or organ, and delivery of the desired molecule can be via diffusion, timed-release bolus, or continuous administration.

A GDF15 molecule can be used to treat, diagnose or ameliorate, a metabolic condition or disorder. In one embodiment, the metabolic disorder is diabetes, e.g., type 2 diabetes. In another embodiment, the metabolic condition or disorder is obesity. In other embodiments, the metabolic condition or disorder is dyslipidemia, elevated glucose levels, elevated insulin levels or diabetic nephropathy. For example, a metabolic condition or disorder that can be treated or ameliorated using a GDF15 molecule includes a state in which a human subject has a fasting blood glucose level of 125 mg/dL or greater, for example 130, 135, 140, 145, 150, 155, 160, 165, 170, 175, 180, 185, 190, 195, 200 or greater than 200 mg/dL. Blood glucose levels can be determined in the fed or fasted state, or at random. The metabolic condition or disorder can also comprise a condition in which a subject is at increased risk of developing a metabolic condition. For a human subject, such conditions include a fasting blood glucose level of 100 mg/dL. Conditions that can be treated using a pharmaceutical composition comprising a GDF15 molecule can also be found in the American Diabetes Association Standards of Medical Care in Diabetes Care 2011, American Diabetes Association, Diabetes Care Vol. 34, No. Supplement 1, S11-S61, 2010.

The administration can be performed such as by intravenous (IV) injection, intraperitoneal (IP) injection, subcutaneous injection, intramuscular injection, or orally in the form of a tablet or liquid formation. A therapeutically effective dose of a GDF15 molecule will depend upon the administration schedule, the unit dose of agent administered, whether the GDF15 molecule is administered in combination with other therapeutic agents, the immune status and the health of the recipient. A therapeutically effective dose is an amount of a GDF15 molecule that elicits a biological or medicinal response in a tissue system, animal, or human being sought by a researcher, medical doctor, or other clinician, which includes alleviation or amelioration of the symptoms of the disease or disorder being treated, *i.e.,* an amount of a GDF15 molecule that supports an observable level of one or more desired biological or medicinal response, for example, lowering blood glucose, insulin, triglyceride, or cholesterol levels; reducing body weight; improving glucose tolerance, energy expenditure, or insulin sensitivity; or reducing food intake. A therapeutically effective dose of a GDF15 molecule can also vary with the desired result.

Illustrated herein is a method comprising measuring a baseline level of one or more metabolically-relevant compounds such as glucose, insulin, cholesterol, lipid in a subject, administering a pharmaceutical composition comprising a GDF15 molecule to the subject, and after a desired period of time, measure the level of the one or more metabolically-relevant compounds (*e.g.*, blood glucose, insulin, cholesterol, lipid) in the subject. The two levels can then be compared to determine the relative change in the metabolically-relevant compound in the subject. Depending on the outcome of that comparison another dose of the pharmaceutical composition can be administered to achieve a desired level of one or more metabolically-relevant compound.

A pharmaceutical composition comprising a GDF15 molecule can be co-administered with another compound or therapeutic agent. A GDF15 molecule (and optionally, its corresponding Fc molecule) can be administered in combination with another therapeutic agent, such as an agent that lowers blood glucose, insulin, triglyceride, or cholesterol levels; lowers body weight; reduces food intake; improves glucose tolerance, energy expenditure, or insulin sensitivity; or any combination thereof (e.g., antidiabetic agent, hypolipidemic agent, anti-obesity agent, anti-hypertensive agent, or agonist of peroxisome proliferator-activator receptor). The identity and properties of a compound to be co-administered with the GDF15 molecule will depend on the nature of the condition to be treated or ameliorated. The agent to be administered with a GDF15 molecule disclosed herein can be a GLP-1R agonist, such as GLP-1 or an analog thereof; or an exendin, exendin analog, or exendin agonist. A nonlimiting list of examples of compounds that can be administered in combination with the pharmaceutical composition include liraglutide, rosiglitizone, pioglitizone, repaglinide, nateglitinide, metformin, exenatide, stiagliptin, pramlintide, glipizide, glimeprirideacarbose, orlistat, lorcaserin, phenterminetopiramate, naltrexonebupropion, setmelanotide, semaglutide, efpeglenatide, canagliflozin, LIK-066, SAR-425899, Tt-401, FGFR4Rx, HDV-biotin and miglitol.

In one embodiment, a GDF15 molecule comprising the amino acid sequence of SEQ ID NO: 50, 52, 54, or 57 is to be administered with another compound or therapeutic agent, such as liraglutide.

In another embodiment, a GDF15 molecule and corresponding Fc molecule comprising the amino acid sequences of SEQ ID NOs: 50 and 36 (C-terminal lysine optional), respectively; SEQ ID NOs: 52 and 36 (C-terminal lysine optional), respectively; SEQ ID NOs: 54 and 36 (C-terminal lysine optional), respectively; or SEQ ID NOs: 57 and 37 (C-terminal lysine optional), respectively; is to be administered with another compound or therapeutic agent, such as liraglutide.

In another embodiment, a GDF15 molecule and corresponding Fc molecule comprising the amino acid sequences of SEQ ID NOs: 50 and 36 (C-terminal lysine optional), respectively, is to be administered with another compound or therapeutic agent, such as liraglutide. In another embodiment, a GDF15 molecule and corresponding Fc molecule comprising the amino acid sequences of SEQ ID NOs: 57 and 37 (C-terminal lysine optional), respectively, is to be administered with another compound or therapeutic agent, such as liraglutide.

A GDF15 molecule to be administered with another therapeutic agent can include concurrent administration of a therapeutically effective amount of the GDF15 molecule (and optionally, its corresponding Fc molecule) and a therapeutically effective amount of the other therapeutic agent. A GDF15 molecule to be administered with another therapeutic agent can include subsequent administration of a therapeutically effective amount of the GDF15 molecule (and optionally, its corresponding Fc molecule) and a therapeutically effective amount of the other therapeutic agent, e.g., administration of a therapeutically effective amount of the GDF15 molecule (and optionally, its corresponding Fc molecule) followed by a therapeutically effective amount of the other therapeutic agent or administration of a therapeutically effective amount of the other therapeutic agent followed by administration of a therapeutically effective amount of the GDF15 molecule (and optionally, its corresponding Fc molecule). Administration of a therapeutically effective amount of the GDF15 molecule (and optionally, its corresponding Fc molecule) can be at least 1, 2, 3, 4, 5, 6, or 7 days after administration of a therapeutically effective amount of the other therapeutic agent. In another embodiment, administration of a therapeutically effective amount of a therapeutically effective amount of the other therapeutic agent can be at least 1, 2, 3, 4, 5, 6, or 7 days after at least 1, 2, 3, 4, 5, 6, or 7 days after administration of a therapeutically effective amount of the GDF15 molecule (and optionally, its corresponding Fc molecule).

A GDF15 molecule to be administered concurrently with another therapeutic agent can comprise administration of a composition comprising both the GDF15 molecule (and optionally its corresponding Fc molecule) and the other therapeutic agent, e.g., a therapeutically effective amount of the GDF15 molecule (and optionally its corresponding Fc molecule) is combined with a therapeutically effective amount of the other agent prior to administration. In another embodiment, concurrent administration of GDF15 molecule (and optionally its corresponding Fc molecule) and another therapeutic agent can comprise concurrent administration of a first composition comprising the GDF15 molecule and a second composition comprising the other therapeutic agent.

In some embodiments, administration of a GDF15 molecule with another therapeutic agent has a synergistic effect. In one embodiment, the effect is greater than the GDF15 molecule (and optionally its corresponding Fc molecule) alone or the other agent. In another embodiment, the effect is greater than an additive effect of both agents (the GDF15 molecule, and optionally its corresponding Fc molecule, plus the other agent). In one embodiment, combination therapy (i.e., administration of a GDF15 molecule, optionally with its corresponding Fc molecule, with another therapeutic agent) has a greater than 1.1, 1.2, 1.3, 1.4, 1.5, 1.6, 1.7, 1.8, 1.9, 2.0, 2.5, 3.0, 3.5, 4.0, 4.5, 5.0, 5.5, 6.0, 6.5, 7.0, 7.5, 8.0, 8.5, 9.0, 9.5, 10, 11, 12, 13, 14, 15, 16, 17, 18, 19, 20, 21, 22, 23, 24, 25,26, 27, 28, 29, or 30 fold effect than GDF15 monotherapy (administration of the GDF15 molecule, and optionally its corresponding Fc molecule). In another embodiment, combination therapy (i.e., administration of a GDF15 molecule, optionally with its corresponding Fc molecule, with another therapeutic agent) has a greater than 1.1, 1.2, 1.3, 1.4, 1.5, 1.6, 1.7, 1.8, 1.9, 2.0, 2.5, 3.0, 3.5, 4.0, 4.5, 5.0, 5.5, 6.0, 6.5, 7.0, 7.5, 8.0, 8.5, 9.0, 9.5, 10, 11, 12, 13, 14, 15, 16, 17, 18, 19, 20, 21, 22, 23, 24, 25, 26, 27, 28, 29, or 30 fold effect than monotherapy with the other agent. The effect can be the amount of body weight lost (e.g., the decrease in total mass or percent body change); the decrease in blood glucose, insulin, triglyceride, or cholesterol levels; the improvement in glucose tolerance, energy expenditure, or insulin sensitivity; or the reduction food intake. The synergistic effect can be about 1, 2, 3, 4, 5, 6, 7, 8, 9, 10, 11, 12, 13, 14, 21, 28, 35, 42, 49, 56, 63, or 70 days after administration.

In one embodiment, a GDF15 molecule and corresponding Fc molecule comprising the amino acid sequences of SEQ ID NOs: 50 and 36 (C-terminal lysine optional), respectively; SEQ ID NOs: 52 and 36 (C-terminal lysine optional), respectively; SEQ ID NOs: 54 and 36 (C-terminal lysine optional), respectively; or SEQ ID NOs: 57 and 37 (C-terminal lysine optional), respectively; to be administered with a GLP-1R agonist (e.g., liraglutide or exendin, or an analog or agonist thereof) has a greater than 1.1, 1.2, 1.3, 1.4, 1.5, 1.6, 1.7, 1.8, 1.9, 2.0, 2.5, 3.0, 3.5, 4.0, 4.5, 5.0, 5.5, 6.0, 6.5, 7.0, 7.5, 8.0, 8.5, 9.0, 9.5, 10, 11, 12, 13, 14, 15, 16, 17, 18, 19, 20, 21, 22, 23, 24, 25,26, 27, 28, 29, or 30 fold effect than GDF15 monotherapy; a greater than 1.1, 1.2, 1.3, 1.4, 1.5, 1.6, 1.7, 1.8, 1.9, 2.0, 2.5, 3.0, 3.5, 4.0, 4.5, 5.0, 5.5, 6.0, 6.5, 7.0, 7.5, 8.0, 8.5, 9.0, 9.5, 10, 11, 12, 13, 14, 15, 16, 17, 18, 19, 20, 21, 22, 23, 24, 25,26, 27, 28, 29, or 30 fold effect than GLP-1R agonist monotherapy (i.e., administration of GLP-1R agonist alone); or both, about 1, 2, 3, 4, 5, 6, 7, 8, 9, 10, 11, 12, 13, 14, 21, 28, 35, 42, 49, 56, 63, or 70 days after administration of the agent(s).

The detailed description and following examples illustrate the present invention and are not to be construed as limiting the present invention thereto. Various changes and modifications can be made by those skilled in the art on the basis of the description of the invention, and such changes and modifications are also included in the present invention.

### EXAMPLES

The following examples, including the experiments conducted and results achieved, are provided for illustrative purposes only and are not to be construed as limiting the present invention.

### Example 1: GDF15(WT)-Linker-Fc Molecules*

* According to the claims, (G₄S)ₙ or (G₄Q)ₙ are envisaged as linkers, wherein n is greater than 0

The GDF15 molecules of scFc-GDF15 (SEQ ID NO: 38) and FcΔ10(-)-(G4S)4-GDF15 (SEQ ID NO: 39) were produced and the activity of the molecules tested.

FcΔ10(-)-(G4S)4-GDF15 (SEQ ID NO: 39) was stably expressed in a serum free, suspension adapted CHO-K1 cell line. It was cloned into a stable expression vector containing puromycin resistance while the Fc chain for forming a heterodimer with FcΔ10(-)-(G4S)4-GDF15, FcΔ10(+,K) (SEQ ID NO: 32), was cloned into a hygromycin containing expression vector (Selexis, Inc.). The plasmids were transfected at a 1:1 ratio using lipofectamine LTX and cells were selected 2 days post transfection in a proprietary growth media containing 10ug/mL puromycin and 600ug/mL hygromycin. Media was exchanged 2 times per week during selection. When cells reached about 90% viability, they were scaled up for a batch production run. Cells were seeded at 2x10⁶/mL in production media. The conditioned medium (CM) produced by the cells was harvested on day 7 and clarified. Endpoint viabilities typically were above 90%.

FcΔ10(-)-(G4S)4-GDF15 (SEQ ID NO: 39) (and any paired Fc) were clarified. Conditioned media was purified using a two-step chromatography procedure. Approximately 5 L of the CM was applied directly to a GE MabSelect SuRe column that had previously been equilibrated with Dulbecco's Phosphate Buffered Saline (PBS). The bound protein underwent three wash steps: first, 3 column volumes (CV) of PBS; next, 1 CV of 20 mM Tris, 100 mM sodium chloride, pH 7.4; and finally, 3 CV of 500 mM L-arginine, pH 7.5. These wash steps remove unbound or lightly bound media components and host cell impurities. The column was then re-equilibrated with 5 CV of 20 mM Tris, 100 mM sodium chloride at pH 7.4 which brought the UV absorbance back to baseline. The desired protein was eluted with 100 mM acetic acid at pH 3.6 and collected in bulk. The protein pool was quickly titrated to within a pH range of 5.0 to 5.5 with 1 M Tris-HCl, pH 9.2. The pH adjusted protein pool was next loaded onto a GE SP Sepharose^{®} HP column that had been previously equilibrated with 20 mM 2-ethanesulfonic acid (MES) at pH 6.0. The bound protein was then washed with 5 CV of equilibration buffer, and finally eluted over a 20 CV, 0 to 50% linear gradient from 0 to 400 mM sodium chloride in 20 mM MES at pH 6.0. Fractions were collected during the elution and analyzed by analytical size-exclusion chromatography (Superdex^{®} 200) to determine the appropriate fractions to pool for a homogeneous product. The SP HP chromatography removes product-related impurities such as free Fc, clipped species, and Fc-GDF15 multimers. The SP HP pool was then buffer exchanged into 10 mM sodium acetate, 5% proline, pH 5.2 by dialysis. It was concentrated to approximately 15 mg/ml using the Sartorius Vivaspin^{®} 20 ten kilo-dalton molecular weight cut-off centrifugal device. Finally, it was sterile filtered and the resulting solution containing the purified Fc-GDF15 molecules was stored at 5° C. Final products were assessed for identity and purity using mass spectral analysis, sodium dodecyl sulfate polyacrylamide electrophoresis and size exclusion high performance liquid chromatography.

ScFc-GDF15 (SEQ ID NO: 38) was produced in a similar manner. This GDF15 molecule was stably expressed in a CHO/CS9 cell line. The molecules were cloned into a stable expression vector. The plasmids (linearized) were transfected at a 1:1 ratio using electroporation and cells were selected 2 days post transfection. Media was exchanged 3 times per week during selection. When cells reached about 90% viability, they were scaled up for a fed batch production run. Cells were seeded at 1x10⁶/mL in production media and fed once when the cell number reached to 4 - 5x10⁶/ml. The conditioned medium (CM) produced by the cells was harvested on day 10 and clarified. Endpoint viabilities typically were above 90%. ScFc-GDF15 was clarified and conditioned media was purified using a two-step coupled chromatography procedure. Conditioned media from multiple harvests were pooled and concentrated nearly 5 fold by ultrafiltration using a 1 sq ft Pellicon^{®} 2 10kD regenerated cellulose membrane (Millipore) by tangential flow filtration. Approximately 5 L of the concentrated CM was applied directly to a GE MabSelect SuRe column that had previously been equilibrated with Dulbecco's Phosphate Buffered Saline (PBS). The non-specifically bound protein was removed by a 12CV PBS wash step. The desired protein was eluted with 0.5% acetic acid at pH 3.5, 150 mM NaCl in 3 CV and collected in a storage loop. The collected protein pool was directly loaded onto a GE HiLoad 26/60 Superdex 200 Prep Grade sizing column that had been previously equilibrated with 30 mM acetate at pH 5.0, 150 mM NaCl. Peak fractions collected during the sizing run were analyzed by sodium dodecyl sulfate polyacrylamide gel electrophoresis to determine the appropriate fractions to pool for a homogeneous product. The pH of the final sizing-pool was adjusted to pH 4.5, with the addition of 10% glacial acetic acid and then buffer exchanged into 10 mM sodium acetate, 9% (w/v) sucrose, pH 4.5 by dialysis. It was concentrated to above 15 mg/ml using a ten kilo-dalton molecular weight cut-off centrifugal device. Protein stability to freezing was tested by 3 cycles of freezing and thawing. Finally, the final lot was sterile filtered and the resulting solution containing the purified GDF15 molecules was stored at -80° C. Final products were assessed for identity and purity using mass spectral analysis, n-terminal sequencing, sodium dodecyl sulfate polyacrylamide electrophoresis and size exclusion high performance liquid chromatography.

Activity of scFc-GDF15 and FcΔ10(-)-(G4S)4-GDF15 was then analyzed for *in vivo* activity. Cynomologus monkeys (n = 10 per group) were administered vehicle, 3 mg/kg of the positive control FGF21-Fc, 1.5 mg/kg of scFc-GDF15, or 1.5 mg/kg of FcΔ10(-)-(G4S)4-GDF15:FcΔ10(+,K) weekly for six weeks, followed by a five-week washout. Body weight and triglyceride levels were determined. Naive male spontaneously obese cynomolgus monkeys were prescreened for health and had a body mass index >41. Monkeys were acclimated to single housing, experimental procedures and handling for 6 weeks prior to treatment. Monkeys were sorted into 4 groups receiving once weekly SC injection for 6 weeks (days 0, 7, 14, 21, 28, and 35) for each group to have similar baseline. Overnight fasting blood samples were collected at pre-dose days -24, -17 and -10, and on days 6, 13, 20, 27, 34, and 41 (6 days after each weekly dose) during the treatment phase. During the washout phase, blood samples were collected on days 48, 55, 62, 69 and 76. Body weight was measured once a week and food intake was monitored daily for each monkey throughout the study. Each monkey received unlimited feed for a limited amount of time (1 hour) at the morning and evening feeding, approximately 8 hours apart. A 150 g apple snack, for a limited amount of time (1 hour), was provided between meals. The remaining food or apple was removed and weighed after each meal or snack to calculate food intake.

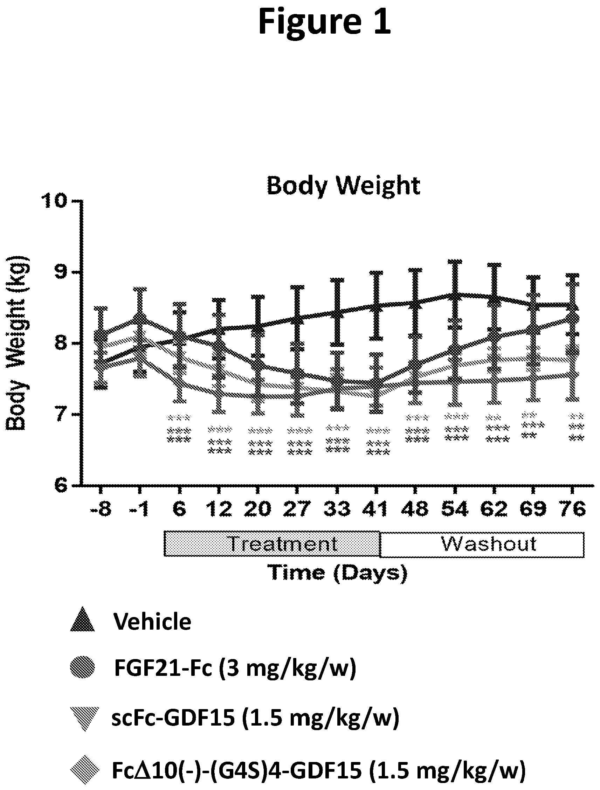

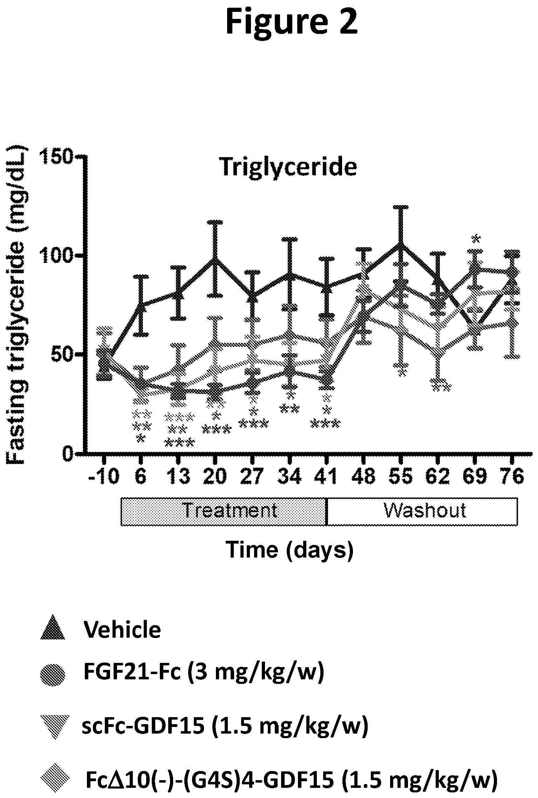

GDF15-Fc fusion proteins reduced body weight **(****Figure 1****)** and triglyceride levels **(****Figure 2****),** similar to FGF21-Fc.

### Example 2: GDF15(WT)-Linker-Fc Molecule Attributes

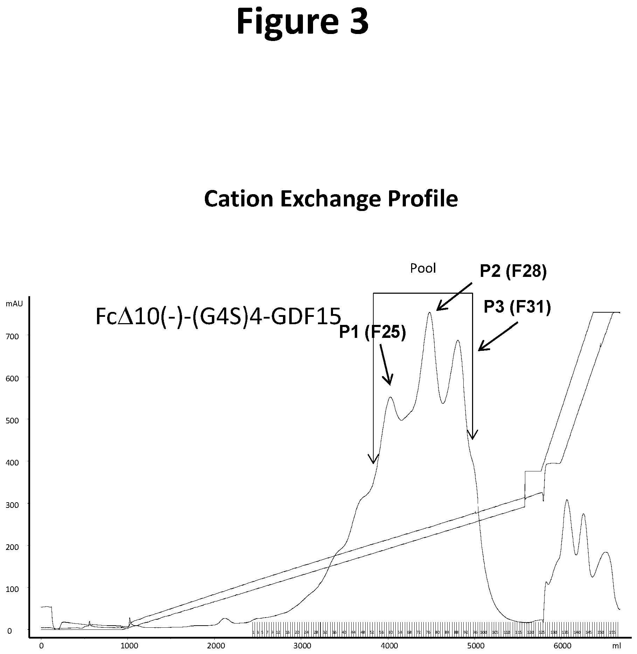

The FcΔ10(-)-(G4S)4-GDF15 (SEQ ID NO: 39) molecule as described in Example 1 and FcΔ10(+)-(G4)-GDF15 (SEQ ID NO: 40) were analyzed for attributes that may affect its stability and manufacturability (e.g., for commercial manufacturing). The GDF15 molecules (e.g., FcΔ10(-)-(G4S)4-GDF15 and FcΔ10(+)-(G4)-GDF15) were determined to be highly heterogeneous (e.g., analysis of an ion exchange column fraction of FcΔ10(-)-(G4S)4-GDF15 shows the molecule is highly heterogeneous, Figure 3), an undesirable feature for manufacturability of a molecule. To determine the attributes of the GDF15 molecules that result in a highly heterogenous population, analysis of the molecules by size exclusion chromatography, SDS PAGE gel, and mass spectrometry was performed. A lack of difference in retention time by size exclusion chromatography indicated that aggregation or gross degradation are unlikely to be responsible for the heterogeneity. There was also a lack of difference on an SDS PAGE gel, which indicated that disulfide mispairing or gross degradation are also unlikely to be responsible for the heterogeneity.

MS analysis was also performed to evaluate the heterogeneity of FcΔ10(-)-(G4S)4-GDF15 (SEQ ID NO: 39). The GDF15 molecule was purified using mono S, 1 ml column and fraction number 25 (P1), fraction number 28 (P2), and fraction number 31 (P3) (Figure 3) were collected and submitted for MS analysis. About 50 µg of the fractions were dried down, resuspended in 25 µL of 150 mM Tris, pH 7.5/ 8M urea/ 40 mM hydroxylamine/10 mM DTT, and then incubated for 1 hour at 37⁰C. The samples were alkylated with 20 mM iodoacetamide (IAM) for 30 minutes at room temperature in the dark. The samples were then diluted to 100 µL with water and 2 µg of trypsin (1:25) and digested overnight at 37⁰C. The digests were acidified, followed by injection onto a Waters (Milford, MA) NanoAcquity UPLC system. Samples were first loaded onto a 180 µm X 20 mm Symmetry C18 trapping column at 15µL/min, followed by peptide separation on an Agilent (Santa Clara, CA) Zorbax 0.5 mm X 250 mm 300SB-C18column. Buffer A was 0.1% formic acid/ water, while buffer B was 0.1% formic acid/ 99.9% acetonitrile. The gradient consisted of initial conditions at 1% B, followed by an increase to 45% B over 85 minutes, to 97% B over 1 minute, isocratic at 97% B for 6 minutes, to 1% B over 3 minutes, and then isocratic at 1% B for 20 minutes. The UPLC column effluent was sprayed into a Thermo Fisher Scientific (San Jose, CA) Orbitrap Velos Pro mass spectrometer using the standard heated electrospray ionization II (HESI II) ionization source. The mass spectrometer method consisted of a full MS scan of *m*/*z [300-*2000] at 30K resolution, followed by MS/MS (CID activation) of the top 10 most abundant precursor ions. The following instrument parameters were used for the analysis: source voltage= 3.5 kV; capillary temperature= 275 ⁰C; S-lens RF level= 50%; activation time= 10 msec; normalized collision energy= 35; isolation width= 2.0 Da; and threshold= 1.0E4. The Xtract component of the Thermo Xcalibur 2.1 software was used for deconvolution of high-resolution MS data. Averaged data from [300-2000] were deconvoluted using a S/N threshold of 1.2 and a resolution of 100,000 at *m*/*z* 400. Deconvoluted peptide masses (glycosylated and non-glycosylated) were displayed as monoisotopic [M+H]⁺. The various glycosylated species were confirmed by the stepwise loss of glycan subunits and the presence of the unglycosylated precursor ion as the most intense fragment following CID activation.

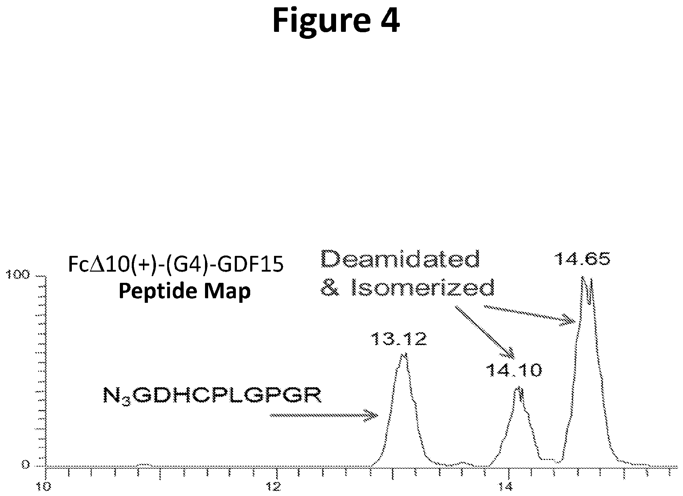

The MS results showed that the varying degrees of deamidated species (e.g., 70% of P1, 47% of P2, and 24% of P3) and glycosylation distribution (mostly monosaccharide and trisaccharide) on the linker contributed to highly heterogeneous nature of the GDF15 molecule as shown in its CEX profile **(****Figure 3****).** It was determined that the (G4S)4 linker *(e.g.,* present in FcΔ10(-)-(G4S)4-GDF15) was highly glycosylated and phosphorylated, with varying degrees and types of glycosylation and/or phosphorylation, and the N-terminus of the active fragment of wildtype human GDF15 was highly susceptible to deamidation and isomerization (see *e.g*., **Figure 4****,** which shows certain masses extracted from the full mass spec data that correspond to the unmodified, deamidated, and isomerized species of the peptide that contains asparagine at position 3. The extracted masses were *m*/*z* [590.25-590.75] from the doubly charged versions of the peptide). The asparagine at position 3 (in reference to SEQ ID NO: 6, the amino acid sequence encoding the active fragment of hGDF15) was highly susceptible to deamidation and isomerization and the aspartate at position 5 (in reference to SEQ ID NO: 6, the amino acid sequence encoding the active fragment of hGDF15) was highly susceptible to isomerization.

Based on these attributes, manufacturing a generally homogenous population of a GDF15-Fc fusion protein having the active fragment of wild type human GDF15 with a linker to the Fc region *(e.g.,* for commercial manufacturing) would be challenging.

### Example 3: Activity of GDF15-Fc Fusion Proteins Without a Linker

To address the heterogeneity issues described in Example 2, new GDF15-Fc fusion proteins that 1) eliminated the linker between the GDF15 region and the Fc region and 2) eliminated or substituted the N-terminal residues of the active fragment of wild-type human GDF15 (e.g., GDF15(Δ3) (SEQ ID NO: 13), where the first three amino acids of the active fragment of wild type human GDF15 is deleted, or GDF15(N3D) (SEQ ID NO: 16), in which the asparagine at position 3 of the active fragment of wild type human GDF15 is mutated to aspartate).

In addition to the charged pair mutation in the Fc region of the GDF15-Fc fusion protein and the Fc molecule for the non-covalent association of the Fc molecule to the Fc region of the GDF15-Fc fusion protein to form a heterodimer, some of the new molecules were designed to also include an interchain disulfide bond in the CH3 region, or "cysteine clamp" (molecules that include "CC" in their designation) to augment the heterodimerization of the GDF-Fc molecule with an Fc molecule.

Four new GDF15-Fc fusion proteins in which 1) the linker between the GDF15 region and the Fc region was deleted and 2) the N-terminal residues of GDF15 were eliminated or substituted were generated. In two of the four molecules, an interchain disulfide bond was also introduced into the CH3 domain of the Fc region of the GDF15-Fc fusion protein (as well as its corresponding Fc molecule for heterodimerization). The potency and pharmacokinetic (PK) properties of these molecules (FcΔ10(-)-GDF15(Δ3) (SEQ ID NO: 41); FcΔ10(-)-GDF15(N3D) (SEQ ID NO: 42); FcΔ10(-,CC)-GDF15(Δ3) (SEQ ID NO: 43); FcΔ10(-,CC)-GDF15(N3D) (SEQ ID NO: 44)) were compared to the earlier generation FcΔ10(-)-(G4S)4-GDF15 (SEQ ID NO: 39), in mice.

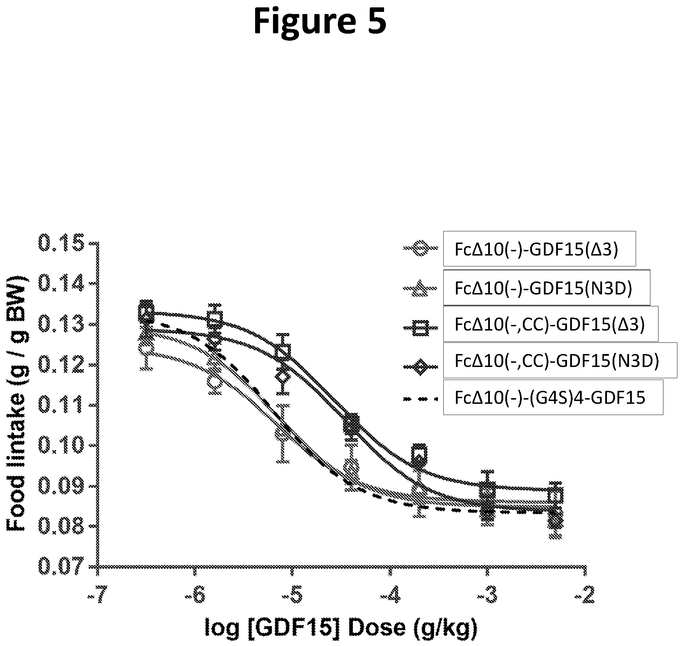

To determine the potency of the molecules, food intake was determined. Seven to eight-week old single-housed male ob/ob mice were sorted into different treatment groups with each group having comparable pretreatment body weight and food intake levels. Animals were treated with 0.32ug/kg, 1.6ug/kg, 8ug/kg, 40ug/kg, 0.2mg/kg, 1mg/kg, or 5mg/kg of a GDF15-Fc fusion protein (a dimer of FcΔ10(-)-GDF15(Δ3):FcΔ10(+,K) (SEQ ID NOs: 41 and 32); FcΔ10(-)-GDF15(N3D):FcΔ10(+,K) (SEQ ID NOs: 42 and 32); or FcΔ10(-,CC)-GDF15(Δ3):FcΔ10(+,K,CC) (SEQ ID NOs: 39 and 32)) through subcutaneous injection, and overnight food intake was measured. Data presented is an average of 2-4 independent studies **(****Figure 5****).** The four new molecules, FcΔ10(-)-GDF15(Δ3) (SEQ ID NO: 41); FcΔ10(-)-GDF15(N3D) (SEQ ID NO: 42); FcΔ10(-,CC)-GDF15(Δ3) (SEQ ID NO: 43); and FcΔ10(-,CC)-GDF15(N3D) (SEQ ID NO: 44), had comparable potency as the earlier generation GDF15-Fc fusion protein, FcΔ10(-)-(G4S)4-GDF15 (SEQ ID NO: 39).

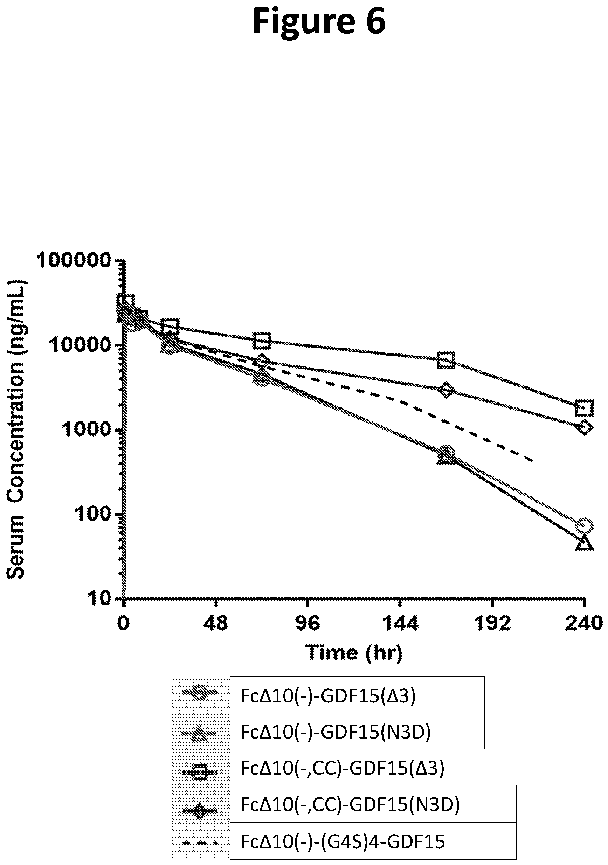

To determine the pharmacokinetics of the molecules, 18-wk old male diet-induced obese C57Bl/6 mice were dosed with 1mg/kg protein subcutaneously, and serial sampling was performed at 1, 4, 8, 24, 72, 168, 240, and 336 hr post-dose. The four new molecules, FcΔ10(-)-GDF15(Δ3) (SEQ ID NO: 41); FcΔ10(-)-GDF15(N3D) (SEQ ID NO: 42); FcΔ10(-,CC)-GDF15(Δ3) (SEQ ID NO: 43); and FcΔ10(-,CC)-GDF15(N3D) (SEQ ID NO: 44), had comparable pharmacokinetic properties as the earlier generation GDF15-Fc fusion protein, FcΔ10(-)-(G4S)4-GDF15 (SEQ ID NO: 39) **(****Figure 6****).**

### Example 4: Further Engineering of GDF15-Fc Fusion Proteins Without a Linker (not claimed)

As the newly designed molecules with improved manufacturability and stability attributes had similar potency and PK properties as the earlier generation molecule, the molecules were further engineered to reduce possible heterogeneity and reduce Fc effector function and increase potency.

To further reduce heterogeneity of the GDF15 region, instead of substituting the asparagine at position 3 with aspartate, the asparagine was substituted with glutamine. In addition, the molecules were engineered to have two changes introduced in the N-terminus of GDF15, *e.g.*, GDF15(Δ3/D5E) (SEQ ID NO: 17), GDF15(N3Q/D5E) (SEQ ID NO: 18) to eliminate the high rate of deamidation and isomerization of the native GDF15 protein. To reduce Fc effector function by and improve potency, the molecules were also engineered to have the hinge region of the Fc region deleted further by having an additional six amino acids deleted from the Fc hinge region *(e.g.,* FcΔ16 instead of FcΔ10) to decrease binding to FcγR. The same engineering of the hinge region was performed for the corresponding Fc molecules to which the GDF15-Fc fusion proteins heterodimerize with.

The activity of the further engineered GDF15-Fc fusion proteins, FcΔ16(-,CC)-GDF15(Δ3/D5E) (SEQ ID NO: 45), FcΔ16(-,CC)-GDF15(N3Q/D5E) (SEQ ID NO: 46), and FcΔ16(-)-GDF15(N3Q/D5E) (SEQ ID NO: 47), were tested in cynomologus monkeys. Naive male spontaneously obese cynomolgus monkeys were acclimated/trained to procedural manipulations (*e.g.*, blood collection, subcutaneous injection, body weight measurement, feeding schedule) for 10 weeks prior to treatment initiation. Eighty (80) monkeys were sorted into 8 treatment groups of n=10 monkeys each based on data collected during acclimation/training phase (blood chemistries and body weight). Each treatment group was administered vehicle, 3 mg/kg of the positive control FGF21-Fc, 0.5 mg/kg of FcΔ16(-,CC)-GDF15(Δ3/D5E) (along with its heterodimerization partner, FcΔ16(+,K,CC) (SEQ ID NO: 35)), 3.0 mg/kg of FcΔ16(-,CC)-GDF15(Δ3/D5E) (along with its heterodimerization partner, FcΔ16(+,K,CC) (SEQ ID NO: 35)), 0.5 mg/kg of FcΔ16(-,CC)-GDF15(N3Q/D5E) (along with its heterodimerization partner, FcΔ16(+,K,CC)), 3.0 mg/kg of FcΔ16(-,CC)-GDF15(N3Q/D5E) (along with its heterodimerization partner, FcΔ16(+,K,CC)), 0.5 mg/kg of FcΔ16(-)-GDF15(N3Q/D5E) (along with its heterodimerization partner, FcΔ16(+,K) (SEQ ID NO: 36)), or 3.0 mg/kg of FcΔ16(-)-GDF15(N3Q/D5E) (along with its heterodimerization partner, FcΔ16(+,K) (SEQ ID NO: 36)). Subcutaneous injections of each were given once a week for 4 weeks during the treatment phase followed by a 4-week washout phase; blood collection and body weight monitoring occurred weekly and food intake occurred daily during treatment and washout phases. The graph represents n=5-6/group and data are represented as group means + SEM. Statistical analysis was performed by ANCOVA and statistical significance is denoted as *p<0.05, **p<0.01 and ***p<0.001 versus vehicle. Monkeys with rapid drug clearance were suspect of anti-drug antibodies (ADAs) and were excluded from analysis.

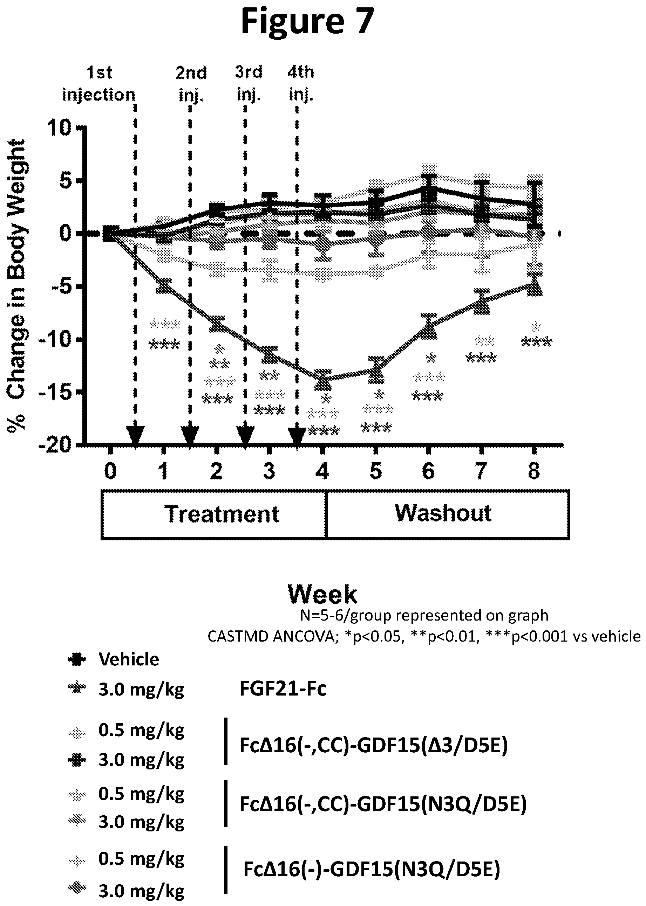

Unexpectedly, the newly engineered GDF15-Fc fusion proteins lost almost all potency **(****Figure 7****).** None of the newly engineered GDF15-Fc fusion proteins reduced body weight to a similar degree as to FGF21-Fc, in contrast to the previously generated GDF15-Fc fusion proteins (see Example 1, **Figure 1****)**

### Example 5: Restoration of GDF15-Fc Fusion Protein Activity in Cynomologus Monkeys

The GDF15-Fc fusion proteins in Example 4 as compared to the GDF15-Fc fusion protein in Example 1 had the following differences as shown in Table 7:

**Table 7: Differences between GDF15-Fc Fusion Proteins in Examples 1 and 4**

| **GDF15-Fc Molecules in Example 1: Efficacious in Cynomologus Monkeys** | **GDF15-Fc Molecules in Example 5: Not Efficacious in Cynomologus Monkeys** |
|---|---|
| Δ10 in Fc region | Δ16 in Fc region |
| No cysteine clamp | Cysteine clamp |
| Has linker | No linker |
| Wild type GDF15 | Two mutations in N-terminus of GDF15 |

To restore potency, different aspects of the molecules that were efficacious in the monkeys were re-introduced into new GDF15-Fc fusion proteins. The cysteine clamp (CH3 interchain disulfide bond) was eliminated and a linker reintroduced for FcΔ16(-)-(G4Q)4-GDF15(N3Q) (SEQ ID NO: 49); FcΔ16(-)-(G4Q)4-GDF15(N3Q/D5E) (SEQ ID NO: 50) and FcΔ16(-)-G4S-GDF15(N3Q/D5E) (SEQ ID NO: 54). However, the linker used in this Example cannot be glycosylated (e.g., G4Q) or was shorter (G4S instead of (G4S)4), to reduce glycosylation. Also, for FcΔ16(-)-(G4Q)4-GDF15(N3Q), the mutation at position 5 was eliminated. Lastly, for the new molecule FcΔ10(-,L234A/L235A)-(G4Q)4-GDF15(N3Q/D5E) (SEQ ID NO: 57), the smaller deletion of the hinge region of the Fc region was reintroduced, however with L234A/L235A mutations in the Fc region, which should eliminate FcγR binding.

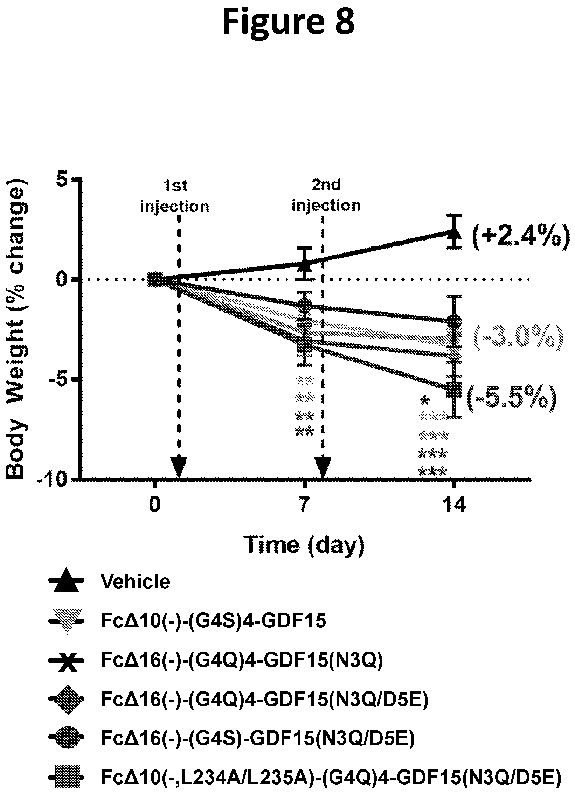

These new molecules were compared to FcΔ10(-)-(G4S)4-GDF15, which was shown to be efficacious in cynomologus monkeys in Example 1. Naive male spontaneously obese cynomolgus monkeys were acclimated/trained to procedural manipulations (e.g., blood collection, subcutaneous injection, body weight measurement, feeding schedule) for 2 weeks prior to treatment initiation. Forty-two (42) monkeys were sorted into 6 treatment groups of n=7 monkeys each based on data collected during acclimation/training phase (blood chemistries and body weight). Each treatment group was administered vehicle, 1.5 mg/kg of FcΔ10(-)-(G4S)4-GDF15 (along with its heterodimerization partner, FcΔ10(+,K)), 1.5 mg/kg of FcΔ16(-)-(G4Q)4-GDF15(N3Q) (along with its heterodimerization partner, FcΔ16(+,K)), 1.5 mg/kg of FcΔ16(-)-(G4Q)4-GDF15(N3Q/D5E) (along with its heterodimerization partner, FcΔ16(+,K)), 1.5 mg/kg of FcΔ16(-)-G4S-GDF15(N3Q/D5E) (along with its heterodimerization partner, FcΔ16(+,K)), or 1.5 mg/kg of FcΔ10(-,L234A/L235A)-(G4Q)4-GDF15(N3Q/D5E) (along with its heterodimerization partner, FcΔ10(+,K,L234A/L235A) (SEQ ID NO: 37). Subcutaneous injections were given once a week for 2 weeks during the treatment phase; blood collection and body weight monitoring occurred weekly and food intake was monitored daily during the treatment phase. The graph represents n=7/group and data is represented as group means + SEM. Statistical analysis was performed by ANCOVA and statistical significance is denoted as *p<0.05, **p<0.01 and ***p<0.001 versus vehicle. The new molecules restored potency **(****Figure 8****).**

Based on these results, the N3Q mutation was determined to not impact the GDF15 activity in the monkeys, and that the double mutation in GDF15 (N3Q/D5E) also did not impact GDF15 activity in the monkeys. The 16-amino acid Fc hinge deletion (Δ16) was also shown to have a similar effect as the 10-amino acid Fc hinge deletion (Δ10) in the monkeys. Lastly, the linker was shown to be a critical component for activity in the monkeys. Though whether the linker is a G4S or G4Q does not affect activity, the length of the linker is important for activity. The longer linkers (e.g., (G4S)4 and (G4Q)4 in **Figure 8****)** are more potent as compared to a shorter linker (e.g., G4S).

### Example 6: Food Intake Assay in ob/ob Mice for FcΔ16(-)-(G4Q)4-GDF15(N3Q/D5E) and FcΔ10(-,L234A/L235A)-(G4Q)4-GDF15(N3Q/D5E)

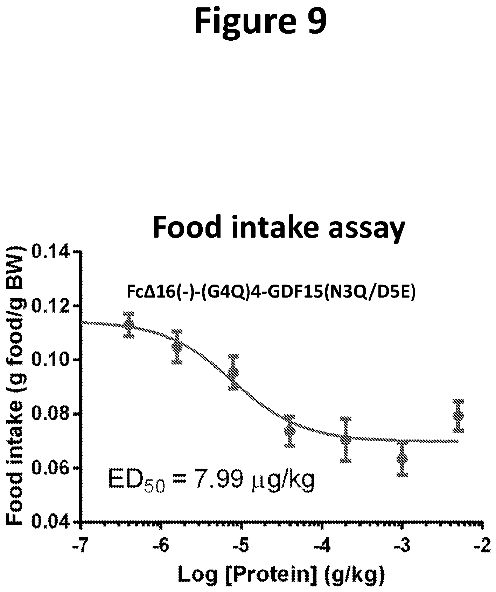

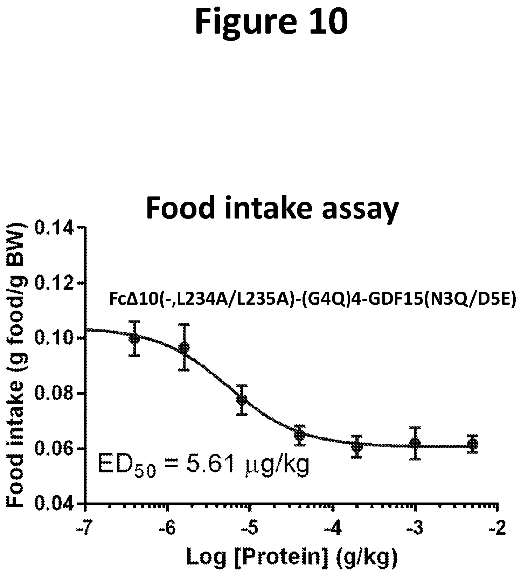

A food intake assay was used to evaluate efficacy of two different GDF15-Fc fusion proteins. Seven to eight weeks-old single-housed male ob/ob mice were sorted into different treatment groups (n=5 per group) with each group having comparable pretreatment body weight and food intake levels. Animals were treated with 0.32ug/kg, 1.6ug/kg, 8ug/kg, 40ug/kg, 0.2mg/kg, 1mg/kg, or 5mg/kg of the heterodimer FcΔ16(-)-(G4Q)4-GDF15(N3Q/D5E):FcΔ16(+,K) or FcΔ10(-,L234A/L235A)-(G4Q)4-GDF15(N3Q/D5E):FcΔ10(+,K,L234A/L235A) through subcutaneous injection, and overnight food intake was measured. The results of a representative experiment for each GDF15-Fc fusion protein is shown in a dose response curve for FcΔ16(-)-(G4Q)4-GDF15(N3Q/D5E) **(****Figure 9****)** and FcΔ10(-,L234A/L235A)-(G4Q)4-GDF15(N3Q/D5E) **(****Figure 10****).** The results show both GDF15-Fc fusion proteins reduce food intake in acute ob/ob mice. The ED50 in this assay is shown in Table 8.

**Table 8: ED50 in Food Intake Assay**

| **Molecule** | **ED50 (mg/kg) n=3-5** |
|---|---|
| FcΔ16(-)-(G4Q)4-GDF15(N3Q/D5E) | 7.4 ±4.2 |
| FcΔ10(-,L234A/L235A)-(G4Q)4-GDF15(N3Q/D5E) | 5.8 ±0.7 |

## Claims

1. A fusion protein comprising a GDF15 region joined to an Fc region via a linker, wherein the GDF15 region comprises the amino acid sequence of SEQ ID NO: 16 or SEQ ID NO: 18, wherein the linker is a (G₄S)ₙ or (G₄Q)ₙ linker, wherein n is greater than 0.

2. The fusion protein of claim 1, which comprises an amino acid sequence of any one of SEQ ID NOs: 50, 52 or 57.

3. The fusion protein of claim 1 or 2, wherein n is 1 or 2.

4. The fusion protein of claim 1 or 2, wherein n is 3 or 4.

5. The fusion protein of any one of claims 1, 3, or 4, wherein the linker is a (G₄Q)ₙ linker.

6. The fusion protein of any one of claims 1, 3, 4, or 5, wherein the Fc region comprises a K392D mutation, K409D mutation, E356K mutation, D399K mutation, or any combination thereof.

7. A dimer or tetramer comprising the fusion protein ofany one of claims 1 to 6.

8. The dimer or tetramer of claim 7, comprising
a) an amino acid sequence of SEQ ID NO: 50 dimerized with an Fc molecule comprising an amino acid sequence of SEQ ID NO: 36, or
b) an amino acid sequence of SEQ ID NO: 57 dimerized with an Fc molecule comprising an amino acid sequence of SEQ ID NO: 37.

9. The fusion protein of any one of claims 1 to 6 or the dimer or tetramer of claim 7 or 8, wherein the Fc region comprises a charged pair mutation, or a truncated hinge region.

10. The fusion protein of any one of claims 1 to 6, wherein the Fc region is selected from the amino acid sequences of SEQ ID NOs: 26-37.

11. The fusion protein of any one of claims 1 to 6, 9, or 10, or the dimer or tetramer of any one of claims 7 to 9, for use in therapy.

12. The fusion protein of any one of claims 1 to 6, 9, or 10, or the dimer or tetramer of any one of claims 7 to 9, for use in a method for treating a metabolic condition or disorder.

13. A pharmaceutical composition comprising the fusion protein of any one of claims 1 to 6, 9, or 10.

14. A pharmaceutical composition comprising the dimer of any one of claims 7 to 9.

15. The pharmaceutical composition of claim 13 or 14, which is for use in treating a metabolic condition or disorder, which is diabetes, obesity, dyslipidemia, elevated glucose levels, elevated insulin levels, or diabetic nephropathy.

16. The pharmaceutical composition for use of claim 15, wherein the metabolic condition or disorder is type 2 diabetes.

17. A pharmaceutical composition comprising a fusion protein of any one of claims 1 to 6, 9, or 10 for use in treating a subject with a fasting blood glucose level of 100 mg/dL or greater.

18. The pharmaceutical composition for use of claim 17, wherein the subject has a fasting blood glucose level of at least 125 mg/dL.

## Patentansprüche

1. Fusionsprotein, umfassend eine GDF15-Region, die über einen Linker mit einer Fc-Region verbunden ist, wobei die GDF15-Region eine Aminosäuresequenz von SEQ ID NO: 16 oder SEQ ID NO: 18 umfasst, wobei der Linker ein (G₄S)ₙ- oder (G₄Q)ₙ-Linker ist, wobei n größer als 0 ist.

2. Fusionsprotein nach Anspruch 1, das eine Aminosäuresequenz einer von SEQ ID NOs: 50, 52 oder 57 umfasst.

3. Fusionsprotein nach Anspruch 1 oder 2, wobei n 1 oder 2 ist.

4. Fusionsprotein nach Anspruch 1 oder 2, wobei n 3 oder 4 ist.

5. Fusionsprotein nach einem der Ansprüche 1, 3 oder 4, wobei der Linker ein (G₄Q)ₙ-Linker ist.

6. Fusionsprotein nach einem der Ansprüche 1, 3, 4 oder 5, wobei die Fc-Region eine K392D-Mutation, K409D-Mutation, E356K-Mutation, D399K-Mutation oder eine Kombination davon umfasst.

7. Dimer oder Tetramer, umfassend das Fusionsprotein nach einem der Ansprüche 1 bis 6.

8. Dimer oder Tetramer nach Anspruch 7, umfassend
a) eine Aminosäuresequenz von SEQ ID NO: 50, die mit einem Fc-Molekül dimerisiert ist, das eine Aminosäuresequenz von SEQ ID NO: 36 umfasst, oder
b) eine Aminosäuresequenz von SEQ ID NO: 57, die mit einem Fc-Molekül dimerisiert ist, das eine Aminosäuresequenz von SEQ ID NO: 37 umfasst.

9. Fusionsprotein nach einem der Ansprüche 1 bis 6 oder Dimer oder Tetramer nach Anspruch 7 oder 8, wobei die Fc-Region eine Mutation eines geladenen Paars oder eine verkürzte Hinge-Region umfasst.

10. Fusionsprotein nach einem der Ansprüche 1 bis 6, wobei die Fc-Region aus den Aminosäuresequenzen von SEQ ID NOs: 26-37 ausgewählt ist.

11. Fusionsprotein nach einem der Ansprüche 1 bis 6, 9 oder 10 oder Dimer oder Tetramer nach einem der Ansprüche 7 bis 9 zur Verwendung in der Therapie.

12. Fusionsprotein nach einem der Ansprüche 1 bis 6, 9 oder 10 oder Dimer oder Tetramer nach einem der Ansprüche 7 bis 9 zur Verwendung in einem Verfahren zur Behandlung eines metabolischen Zustands oder einer metabolischen Störung.

13. Pharmazeutische Zusammensetzung, umfassend das Fusionsprotein nach einem der Ansprüche 1 bis 6, 9 oder 10.

14. Pharmazeutische Zusammensetzung, umfassend das Dimer nach einem der Ansprüche 7 bis 9.

15. Pharmazeutische Zusammensetzung nach Anspruch 13 oder 14 zur Verwendung bei der Behandlung eines metabolischen Zustands oder einer metabolischen Störung, der bzw. die Diabetes, Adipositas, Dyslipidämie, erhöhte Glucosespiegel, erhöhte Insulinspiegel oder diabetische Nephropathie ist.

16. Pharmazeutische Zusammensetzung zur Verwendung nach Anspruch 15, wobei der metabolische Zustand oder die metabolische Störung Typ-2-Diabetes ist.

17. Pharmazeutische Zusammensetzung, umfassend ein Fusionsprotein nach einem der Ansprüche 1 bis 6, 9 oder 10 zur Verwendung bei der Behandlung eines Subjekts mit einem Nüchternblutglucosespiegel von 100 mg/dL oder höher.

18. Pharmazeutische Zusammensetzung zur Verwendung nach Anspruch 17, wobei das Subjekt einen Nüchternblutglucosespiegel von mindestens 125 mg/dL aufweist.

## Revendications

1. Protéine de fusion comprenant une région GDF15 reliée à une région Fc via un lieur, dans laquelle la région GDF15 comprend une séquence d'acides aminés de SEQ ID NO: 16 ou SEQ ID NO: 18, dans laquelle le lieur est un lieur (G₄S)ₙ ou (G₄Q)ₙ, dans laquelle n est supérieur à 0.

2. Protéine de fusion selon la revendication 1, qui comprend une séquence d'acides aminés de l'une quelconque des SEQ ID NOs: 50, 52 ou 57.

3. Protéine de fusion selon la revendication 1 ou 2, dans laquelle n est 1 ou 2.

4. Protéine de fusion selon la revendication 1 ou 2, dans laquelle n est 3 ou 4.

5. Protéine de fusion selon l'une quelconque des revendications 1, 3 ou 4, dans laquelle le lieur est un lieur (G₄Q)ₙ.

6. Protéine de fusion selon l'une quelconque des revendications 1, 3, 4 ou 5, dans laquelle la région Fc comprend une mutation K392D, une mutation K409D, une mutation E356K, une mutation D399K, ou une combinaison de celles-ci.

7. Dimère ou tétramère comprenant la protéine de fusion selon l'une quelconque des revendications 1 à 6.

8. Dimère ou tétramère selon la revendication 7, comprenant :
a) une séquence d'acides aminés de SEQ ID NO: 50 dimérisée avec une molécule Fc comprenant une séquence d'acides aminés de SEQ ID NO: 36, ou
b) une séquence d'acides aminés de SEQ ID NO: 57 dimérisée avec une molécule Fc comprenant une séquence d'acides aminés de SEQ ID NO: 37.

9. Protéine de fusion selon l'une quelconque des revendications 1 à 6 ou dimère ou tétramère selon la revendication 7 ou 8, dans laquelle la région Fc comprend une mutation d'une paire chargée ou une région charnière tronquée.

10. Protéine de fusion selon l'une quelconque des revendications 1 à 6, dans laquelle la région Fc est choisie parmi les séquences d'acides aminés des SEQ **ID** NOs: 26-37.

11. Protéine de fusion selon l'une quelconque des revendications 1 à 6, 9 ou 10, ou dimère ou tétramère selon l'une quelconque des revendications 7 à 9, pour utilisation en thérapie.

12. Protéine de fusion selon l'une quelconque des revendications 1 à 6, 9 ou 10, ou dimère ou tétramère selon l'une quelconque des revendications 7 à 9, pour utilisation dans un procédé de traitement d'une affection ou d'un trouble métabolique.

13. Composition pharmaceutique comprenant la protéine de fusion selon l'une quelconque des revendications 1 à 6, 9 ou 10.

14. Composition pharmaceutique comprenant le dimère selon l'une quelconque des revendications 7 à 9.

15. Composition pharmaceutique selon la revendication 13 ou 14 pour utilisation dans le traitement d'une affection ou d'un trouble métabolique qui est le diabète, l'obésité, la dyslipidémie, des taux de glucose élevés, des taux d'insuline élevés ou une néphropathie diabétique.

16. Composition pharmaceutique pour utilisation selon la revendication 15, dans laquelle l'affection ou le trouble métabolique est le diabète de type 2.

17. Composition pharmaceutique comprenant une protéine de fusion selon l'une quelconque des revendications 1 à 6, 9 ou 10 pour utilisation dans le traitement d'un sujet ayant un taux de glucose sanguin à jeun de 100 mg/dL ou plus.

18. Composition pharmaceutique pour utilisation selon la revendication 17, dans laquelle le sujet a un taux de glucose sanguin à jeun d'au moins 125 mg/dL.
